# EUROPEAN PATENT SPECIFICATION

(11) **EP 3 917 608 B1**
(45) Date of publication and mention of the grant of the patent: **04.06.2025**
(21) Application number: 20740420.3
(22) Date of filing: 29.01.2020
(51) Int. Cl.: A61M 37/00, A61M 25/00

(54) **A MICRONEEDLE DEVICE AND A MICRONEEDLE DEVICE FOR USE IN A METHOD FOR THE TREATMENT OF A LUMEN, VESSEL, CAVITY OR A FISTULA**
MIKRONADELVORRICHTUNG UND MIKRONADELVORRICHTUNG ZUR VERWENDUNG IN EINEM VERFAHREN ZUR BEHANDLUNG EINES LUMENS, EINES GEFÄSSES, EINES HOHLRAUMS ODER EINER FISTEL
DISPOSITIF À MICRO-AIGUILLES ET DISPOSITIF À MICRO-AIGUILLES DESTINÉ À ÊTRE UTILISÉ DANS UNE MÉTHODE DE TRAITEMENT D'UNE LUMIÈRE, D'UN VAISSEAU, D'UNE CAVITÉ OU D'UNE FISTULE

(30) Priority: 29.01.2019 IE S20190014
(43) Date of publication of application: 08.12.2021
(73) Proprietor: ACJ Hawthorns Unlimited Company, Galway (IE)
(72) Inventor: O'DEA, John, Galway (IE)
(74) Representative: Gorman, Francis Fergus
(86) International application number: PCT/IE2020/000003
(87) International publication number: WO 2020/157740

(56) References cited:
- EP-A2- 2 777 737
- WO-A1-97/02859
- US-A1- 2009 118 662
- US-A1- 2011 264 048

## Description

The present invention relates to a microneedle device, and the invention also relates to a microneedle device for use in a method for the treatment of a lumen, vessel, cavity, organ or muscular organ with a therapeutic substance, for example, a therapeutic fluid, such as a therapeutic liquid, or a therapeutic liquid or fluid comprising stem cells, drugs or other therapeutic substances, as well as microbeads or other such bulking agents and substances. The invention also relates to a microneedle device for use in a method for the treatment of a fistula, and in particular, though not limited to a microneedle device for use in a method for the treatment of a fistula with a therapeutic substance, for example, a therapeutic fluid, such as, for example, a therapeutic fluid comprising stem cells, for example, a therapeutic liquid comprising stem cells, and the invention also relates to a microneedle device for use in a method for the treatment of an anal fistula with a therapeutic fluid, for example, a therapeutic liquid or fluid comprising stem cells.

The term fistula is a medical term for an infected tunnel that develops between an infected gland and the skin of a subject, or between two adjacent vessels in a subject. For example, an anal fistula is an infected tunnel which develops between the skin and the muscular opening at the end of the digestive tract, namely, the anus. In general, most anal fistulae are the result of an infection, which in general starts in an anal gland. This infection results in an abscess that drains spontaneously, or is drained surgically through the skin next to the anus. The fistula forms a tunnel under the skin and connects with the infected gland.

Recently, new regenerative stem cell therapies have been developed which can promote inward tissue growth into a fistula which seals off the fistula, thereby preventing ongoing infection, and the need to continue cleaning the fistula. The stem cells are injected adjacent various sections of the fistula. The injection of the stem cells into the fistula, in general, is carried out with a needle and syringe. This requires urging the needle through the fistula and endeavouring to inject the stem cells at spaced apart locations along the fistula into the tissue adjacent the fistula. It is critical that the stem cells should be injected to a depth of no more than 1 mm to 2mm below the surface of the fistula in order to promote growth and infill of tissue into the fistula. A problem with this technique is that once the needle of the syringe needle assembly is entered into the fistula, it is no longer possible to determine when the pointed tip of the needle is within the fistula, or has been urged from the fistula into tissue adjacent the fistula, and in particular, it is impossible to determine the depth to which the tip of the needle has been inserted into the tissue from the fistula.

Additionally, in the treatment of a lumen, vessel, cavity, organ or muscular organ, in a human or animal subject, for example, in the treatment of the oesophagus, where the oesophagus or other lumen, vessel, cavity, organ or muscular organ, is being treated by injecting a therapeutic fluid or liquid into the wall or tissue of the oesophagus, lumen, vessel, cavity, organ or muscular organ, it is important that the needle or other injecting device does not extend completely through the wall or tissue of the oesophagus, lumen, vessel, cavity, organ or muscular organ, but rather the needle or other injecting device should be inserted into the wall or tissue of the oesophagus, lumen, vessel, cavity, organ or muscular organ to a depth such that the tip of the needle should terminate within the wall or tissue of the oesophagus, lumen, vessel, cavity, organ or muscular organ. Otherwise, should the needle extend completely through the wall or tissue of the oesophagus, lumen, vessel, cavity, organ or muscular organ, the injected fluid or liquid would be injected into a cavity in which the oesophagus, lumen, vessel, cavity, organ or muscular organ is located, or into an organ adjacent the oesophagus, lumen, vessel, cavity, organ or muscular organ, with, in some cases, possibly fatal consequences. Since the oesophagus, and the lower oesophageal sphincter, can only be accessed either orally or nasally through an endoscope or a catheter, it is virtually impossible with a conventional needle to control the depth to which a needle is inserted into the wall or tissue of the oesophagus or the lower oesophageal sphincter. This is also a problem in the treatment of the walls or tissue of other lumen, vessels, cavities, organs and muscular organs, such as sphincters in the human or animal body with an injectable fluid or liquid where such lumen, vessels, cavities, organs and muscular organs are not easily accessible in the human or animal body.

U.S. Patent Application Specification No. 2009/0118662 of Schnall discloses a drug delivery device for delivering a liquid drug to a site in a lumen or vessel to be treated. The drug delivery device comprises an elongated catheter having an inflatable balloon located adjacent a distal end thereof and extending around the catheter. A plurality of drug delivery units are located circumferentially around the balloon. Each drug delivery unit comprises a plurality of microneedles arranged to extend transversely relative to the catheter, and a rupturable capsule located between the microneedles and the balloon containing the drug. As the balloon is inflated the microneedles are urged through an outer compressible sponge layer and into engagement with the site to be treated in the lumen or vessel. As further pressure is progressively applied by the inflating balloon to the rupturable capsule, the capsule bursts, thereby allowing the drug contained therein to be urged through the microneedles to the site to be treated.

PCT Specification No. WO 97/02859 of Leonhardt discloses an intra-cavity therapeutic agent delivery device which comprises an elongated catheter having located therein a plurality of needle assemblies mounted circumferentially around an inflatable balloon. Each needle assembly comprises a hollow shaft extending through the catheter to the proximal end thereof for receiving the drug to be delivered to the site. Elasticated bands extending around the needle assemblies are configured to urge the needle assemblies inwardly into and to retain the needle assemblies within the catheter. Radial extending bores through the catheter accommodate the needles of the needle assembly through the catheter on inflating of the balloon for engaging the site to be treated. On deflating the balloon the bands urge the needles inwardly into the catheter.

U.S. Patent Application Specification No. 2011/0264048 of O'Dea et al. discloses a microneedle delivery device which comprises a plurality of microneedles in communication with respective drug accommodating chambers accommodating a drug to be delivered to a site to be treated. A drive substance accommodating chamber corresponding to each drug chamber is separated by an expandable membrane from the drug chamber. Each drive substance chamber stores a drive substance, which when activated expands for urging the membrane into the drug chamber, for in turn urging the drug from the drug chamber through the microneedle.

European Patent Specification No. 2,777,737 of Braga discloses a fluid delivery catheter for delivering fluid to a site in a lumen or vessel. The delivery catheter comprises a plurality of microneedles located in wells formed into the catheter. The wells are urgeable from a retracted state with the microneedles retracted within the wells to an outwardly extended state with the microneedles extending radially outwardly from the catheter for engaging the site to be treated. The wells are urged from the retracted state to the extended state in response to the injectable fluid being urged into the catheter under pressure, and the injectable fluid is then in turn urged through the microneedles to the site in the lumen or vessel.

There is therefore a need for a device which addresses at least some of these problems. There is also a need for a device for use in a method for the treatment of a lumen, vessel, cavity, fistula, organ or muscular organ which addresses at least some of these problems.

The present invention is directed towards a microneedle device and to a microneedle device for use in a method for the treatment of a lumen, vessel, cavity, organ or muscular organ with a therapeutic substance, for example, a therapeutic fluid, such as a therapeutic liquid, or a therapeutic liquid or fluid comprising stem cells, drugs or other therapeutic substances, as well as microbeads and other such bulking agents and substances. The present invention is also directed towards a microneedle device and to a microneedle device for use in a method for the treatment of a fistula, for example, an anal fistula, and in particular, to a microneedle device for use in a method for the treatment of a fistula with a therapeutic fluid, such as a therapeutic fluid comprising stem cells, such as a therapeutic liquid comprising stem cells, or a therapeutic fluid or liquid comprising drugs or other such substances. The present invention is also directed towards a microneedle device for use in a method for the treatment of a wall or tissue of a lumen, vessel, cavity, organ or muscular organ by injecting a fluid into the wall, tissue or other bodily material of the wall of the lumen, vessel, cavity, organ or muscular organ in a human or animal subject.

According to the invention there is provided a microneedle device comprising a carrier element defining a longitudinally extending main axis, at least one microneedle defining a needle axis, the at least one microneedle being carried on the carrier element with the needle axis extending substantially transversely relative to the main axis defined by the carrier element, and an operating means co-operable with one of the carrier element and the at least one microneedle for urging the microneedle transversely outwardly relative to the main axis from a rest state to an engagement state for engaging tissue, wherein one of the carrier element and the operating means is configured to accommodate a communicating means for communicating each microneedle with a source of the fluid to be delivered to a subject through the microneedle device, and the communicating means comprises at least one main chamber located in the one of the carrier element and the operating means, each main chamber being configured to accommodate the fluid to be delivered to a subject and to communicate with the corresponding one or more of the microneedles.

In one embodiment of the invention the operating means is co-operable with the carrier element for urging the at least one microneedle transversely outwardly relative to the main axis.

In another embodiment of the invention the carrier element defines a main bore extending longitudinally into the carrier element from a proximal end thereof. Preferably, the main bore defined by the carrier element extends substantially centrally in the carrier element parallel to the main axis thereof. Advantageously, the operating means is engageable in the main bore defined by the carrier element.

In another embodiment of the invention the operating means is configured for increasing the cross-section of the main bore defined by the carrier element. Preferably, the operating means is provided with a transverse cross-sectional dimension greater than a corresponding transverse cross-sectional dimension of the main bore defined by the carrier element. Advantageously, the external transverse cross-section of the operating means is greater than the transverse cross section of the main bore defined by the carrier element. Preferably, the shape of the transverse cross-section of the operating means is substantially similar to the shape of the transverse cross-section of the main bore defined by the carrier element.

In one embodiment of the invention the operating means comprises an operating member, the operating member being of non-deformable transverse cross-section.

In another embodiment of the invention the operating means comprises an expandable member of expandable transverse cross-section. Preferably, the operating means comprises an inflatable element. Advantageously, the operating means comprises an inflatable balloon. Ideally, the inflatable balloon is located on a catheter having an inflating medium accommodating bore extending therethrough for accommodating an inflating medium for inflating the balloon.

In one embodiment of the invention the expandable element comprises a balloon catheter.

In another embodiment of the invention the main bore defined by the carrier element is of substantially circular transverse cross-section.

Preferably, the operating means is of substantially circular external transverse cross-section.

In one embodiment of the invention the carrier element is of substantially circular external transverse cross-section.

In another embodiment of the invention the carrier element comprises a pair of shells.

In another embodiment of the invention the shells of the carrier element define the main bore therebetween. Preferably, the shells are resiliently urged towards each other. Advantageously, the shells are resiliently urged towards each other into a rest state thereof for retaining the at least one microneedle in the rest state thereof.

In one embodiment of the invention the shells of the carrier element in the rest state define the main bore.

In another embodiment of the invention the shells are resiliently urged into the rest state by at least one band extending around the shells, the at least one band comprising a resilient material. Preferably, a plurality of the resilient bands are located around the shells at longitudinally spaced apart intervals along the shells.

In another embodiment of the invention the shells are urgeable radially outwardly relative to the main axis, against the resilient urging of the shells into the rest state, from the rest state to an engagement state by the operating means for urging the at least one microneedle transversely outwardly relative to the main axis from the rest state thereof to the engagement state thereof. Preferably, the shells are resiliently urged into sealing engagement with the operating means by the at least one band at least when the shells are in the engagement state thereof.

In one embodiment of the invention at least one microneedle is located on each shell.

Preferably, each microneedle defines a fluid accommodating bore extending therethrough. Advantageously, the fluid accommodating bore extending through each microneedle extends centrally through the microneedle or is offset from the needle axis.

Preferably, the main chamber is configured to accommodate fluid passing therethrough from a fluid source to the one or more of the microneedles. Advantageously, the main chamber is configured to communicate with a fluid delivery means configured to deliver a fluid from a remote source to the main chamber.

In one embodiment of the invention the fluid delivery means comprises an elongated fluid delivery needle having a fluid accommodating bore extending therethrough to a distal end thereof. Preferably, the fluid accommodating bore extending through the fluid delivery needle terminates adjacent the distal end thereof in a sidewardly facing outlet port spaced apart slightly proximally from the distal end of the fluid delivery needle. Advantageously, the fluid delivery needle terminates at its distal end in a distal pointed tip.

In one embodiment of the invention the one of the carrier element and the operating means comprises a fluid delivery needle accommodating duct for accommodating the fluid delivery needle therethrough to the main chamber.

In another embodiment of the invention the fluid delivery needle accommodating duct extends longitudinally through the one of the carrier element and the operating means. Preferably, the fluid delivery needle accommodating duct extends through the one of the carrier element and the operating means in which the main chamber is located.

In one embodiment of the invention a plurality of the main chambers are provided, and the fluid delivery needle accommodating duct is configured to communicate with each of the main chambers. Preferably, the fluid delivery needle accommodating duct is configured to accommodate the fluid delivery needle sequentially to the main chambers.

In one embodiment of the invention each main chamber is isolated from the fluid delivery accommodating duct by a first sealing element. Preferably, each first sealing element is configured to sealably accommodate the fluid delivery needle therethrough to the corresponding one of the main chambers. Advantageously, each first sealing element comprises a self-resealing sealing element configured to reseal itself on withdrawal of the fluid delivery needle.

In one embodiment of the invention each main chamber is configured to store a fluid to be delivered to the subject.

In another embodiment of the invention each main chamber comprises an urging means co-operable with the main chamber for urging a fluid in the main chamber from the main chamber through the corresponding one or more of the microneedles. Preferably, each urging means is urgeable into the corresponding main chamber for urging the fluid therefrom.

In another embodiment of the invention a secondary chamber is provided corresponding to each main chamber, each secondary chamber being co-operable with the urging means of the corresponding main chamber, and being configured to accommodate a drive medium for urging the urging means into the corresponding main chamber. Preferably, each main chamber is isolated from the corresponding secondary chamber by the corresponding urging means.

In one embodiment of the invention each urging means comprises a main membrane. Preferably, each main membrane comprises a flexible membrane. Advantageously, each main membrane comprises an elastically expandable membrane.

In another embodiment of the invention each main chamber is isolated from the corresponding one or more of the microneedles by a secondary rupturable membrane. Preferably, the secondary membrane comprises a resilient material. Advantageously, the secondary membrane is rupturable in response to the urging means of the corresponding main chamber being urged into the main chamber.

In one embodiment of the invention the drive medium comprises an expandable medium.

Preferably, each secondary chamber comprises an activating means, and the drive medium is expandable in response to activation of the activating means. Advantageously, each activating means comprises a heating means located in or adjacent the corresponding secondary chamber. Preferably, each heating means comprises an electrically powered heating means powered through an electrically conductive means extending through the carrier element or the operating means.

In one embodiment of the invention the drive medium comprises microbeads of the type which expand to a multiple of their size when heated.

In another embodiment of the invention a drive medium delivery needle accommodating duct is provided for accommodating a drive medium delivery needle to the one or more secondary chambers for delivering the drive medium to the one or more secondary chambers.

In another embodiment of the invention each secondary chamber is isolated from the drive medium delivery needle accommodating duct by a second sealing element. Advantageously, the second sealing element comprises a self-resealing sealing element. Preferably, the second sealing element is configured to sealably accommodate the drive medium delivery needle therethrough, and is configured to reseal itself on withdrawal of the drive medium delivery needle.

In one embodiment of the invention the drive medium delivery needle comprises an elongated bore extending therethrough and terminating in an outlet port adjacent the distal end thereof. Preferably, the distal outlet port of the drive medium delivery needle is located on the side of the drive medium delivery needle adjacent but spaced apart proximally from the distal end of the drive medium delivery needle, and the drive medium delivery needle terminates in a distal pointed tip.

In one embodiment of the invention the drive medium comprises a pressurised fluid. Preferably, the drive medium comprises a pressurised gas. Advantageously, the drive medium comprises pressurised air.

In one embodiment of the invention the drive medium delivery needle accommodating duct extends through the carrier element or the operating means.

In another embodiment of the invention the drive medium needle accommodating duct is located in the one of the carrier element and the operating means in which the secondary chamber is located.

In one embodiment of the invention each secondary chamber is located in the carrier means.

In another embodiment of the invention each secondary chamber is located in the operating means.

In another embodiment of the invention each secondary chamber is located in the one of the carrier element and the operating means in which the corresponding one of the main chambers is located.

In another embodiment of the invention each main chamber is located in the carrier element.

In another embodiment of the invention each main chamber is located in the operating means.

Preferably, the operating means is alignable with the carrier element.

In another embodiment of the invention the operating means is alignable with the carrier element for aligning the at least one main chamber with a corresponding one or more of the microneedles for communicating the main chamber with the fluid accommodating bore of each microneedle corresponding to the main chamber.

In another embodiment of the invention the operating means is alignable longitudinally with the carrier element.

In a further embodiment of the invention complementary longitudinal alignment means are provided on the carrier element and the operating means for aligning the operating means longitudinally relative to the carrier element.

In another embodiment of the invention the operating means is circumferentially alignable with the carrier element.

In one embodiment of the invention complementary circumferential alignment means are provided on the carrier element and the operating means for circumferentially aligning the operating means with the carrier element.

In one embodiment of the invention the microneedle device further comprises a shielding means for protecting the microneedles.

In one embodiment of the invention the shielding means comprises a tubular shield having a carrier element accommodating bore extending therethrough for accommodating the carrier element therein.

In another embodiment of the invention the shielding means comprises an elongated tubular shield.

In another embodiment of the invention the tubular shield is configured to accommodate the carrier element in the carrier element accommodating bore with the main axis defined by the carrier element extending substantially parallel to the tubular shield.

Preferably, the carrier element accommodating bore is configured to accommodate the carrier element and the microneedles therein when the microneedles are in the rest state.

Advantageously, the tubular shield is configured for permitting insertion and withdrawal of the carrier element into and out of the carrier element accommodating bore through at least one end of the tubular shield.

In one embodiment of the invention the tubular shield is of length at least equal to the length of the carrier element. Preferably, the tubular shield is of length greater than the carrier element.

Preferably, the shielding means comprises an elongated tubular shield extending between a proximal end and a distal end, the distal end thereof being configured to extend to a site to be treated.

In one embodiment of the invention the proximal end of the tubular shield is configured to extend exteriorly of the subject.

In one embodiment of the invention the tubular shield is of length sufficient to extend from the site to be treated to a location exteriorly of the subject.

Preferably, the carrier element accommodating bore of the tubular shield is located at least adjacent the distal end thereof.

Advantageously, the tubular shield is provided with an elongated bore extending from the carrier element accommodating bore to the proximal end of the tubular shield for accommodating the operating means therethrough to the carrier element.

In one embodiment of the invention the shielding means comprises a microneedle accommodating bore extending through the shielding means for accommodating a corresponding one of the microneedles therethrough.

Preferably, the microneedle accommodating bore extends substantially radially through the shielding means.

Advantageously, one of the shielding means and the carrier element is moveable transversely relative to the other one of the shielding means and the carrier element for urging the at least one microneedle through the corresponding microneedle accommodating bore in the shielding means.

In one embodiment of the invention the carrier element is moveable from the rest state to the engagement state for urging the corresponding one of the microneedles from the rest state within the shielding means to the engagement state extending outwardly from the shielding means for engaging tissue to be injected.

Additionally, the invention provides the microneedle device according to the invention for use in a method for the treatment of a site in a human or animal body with an injectable fluid.

The invention also provides a microneedle device according to the invention for use in a method for the treatment of a site in a human or animal body by a regenerative stem cell therapy.

Further the invention provides a microneedle device according to the invention for use in a method for the treatment of a site in a lumen, vessel, cavity, organ or muscular organ in a human or animal body by a regenerative stem cell therapy.

The invention also provides a microneedle device according to the invention for use in a method for the treatment of a fistula in a human or animal body by a regenerative stem cell therapy.

In one embodiment of the invention the microneedle device is configured to inject a therapeutic fluid into tissue adjacent the site or within the fistula.

In another embodiment of the invention the microneedle device is configured to inject a therapeutic fluid comprising stem cells into tissue adjacent the site or within the fistula.

In another embodiment of the invention the microneedle device is configured to inject a therapeutic liquid comprising stem cells entrained therein into tissue adjacent the site or within the fistula.

Further the invention provides a microneedle device for use in a method for the treatment of a fistula with a therapeutic fluid, the microneedle device comprising a microneedle device according to the invention.

The invention also provides a microneedle device for use in a method for the treatment of an anal fistula with a therapeutic fluid, the microneedle device comprising a microneedle device according to the invention.

Further the invention provides a microneedle device for use in a method for the treatment of a fistula in a human or animal body by a regenerative stem cell therapy, the microneedle device comprising a microneedle device according to the invention.

Further the invention provides a microneedle device for use in a method for the treatment of an anal fistula in a human or animal body by a regenerative stem cell therapy, the microneedle device comprising a microneedle device according to the invention.

In one embodiment of the invention a therapeutic fluid comprising stem cells is injected into tissue of the fistula through the at least one microneedle of the microneedle device.

The invention also provides a microneedle device for use in a method for the treatment of a fistula in a human or animal body with a drug, the microneedle device comprising a microneedle device according to the invention.

The invention also provides a microneedle device for use in the treatment of a site in a lumen, vessel, cavity, organ or muscular organ in a human or animal body with one or more of a therapeutic fluid, a drug, microbeads or a bulking agent, the microneedle device comprising a microneedle device according to the invention.

The invention also provides a microneedle device for use in a method for the treatment of a site in a lumen, vessel, cavity, organ or muscular organ in a human or animal body by regenerative stem cell therapy, the microneedle device comprising a microneedle device according to the invention.

In one embodiment of the invention a therapeutic fluid comprising stem cells is injected into a wall or tissue of the lumen, vessel, cavity, organ or muscular organ adjacent the site thereof to be treated through the at least one microneedle of the microneedle device.

The advantages of the microneedle devices according to the invention are many. A particularly important advantage of the microneedle devices according to the invention is that the microneedle devices ensure that the therapeutic fluid or liquid is delivered precisely to the desired depth in the tissue, in cases where the microneedle devices are used to deliver the therapeutic fluid or liquid to tissue in a fistula, and in the tissue, the wall or musculature of a lumen, vessel, cavity, organ or muscular organ in cases where the microneedle devices are used to deliver the therapeutic fluid or liquid to a site in the lumen, vessel, cavity, organ or muscular organ. This is a particularly important advantage in the case of a fistula, since it is essential that the therapeutic fluid or liquid should be delivered into the tissue adjacent the fistula to a precise depth, typically, in the order of 1mm to 2mm. This is also a particularly important advantage in the treatment of a site in a lumen, vessel, cavity, organ or muscular organ, since once the therapeutic liquid or fluid is delivered to a precise depth in the wall of the lumen, vessel, cavity, organ or muscular organ, there is no danger of the therapeutic fluid or liquid being delivered into an adjacent cavity in which the lumen, vessel, cavity, organ or muscular organ is located, or into an organ adjacent the lumen, vessel, cavity, organ or muscular organ the site of which is being treated, which could otherwise happen if the depth in tissue, wall or musculature of the lumen, vessel, cavity, organ or muscular organ at which the therapeutic liquid or fluid is being delivered, was not accurately controlled.

A further advantage of the invention is that the microneedle devices according to the invention allow a precise dose of the therapeutic fluid or liquid to be delivered to the site of a lumen, vessel, cavity, organ or muscular organ to be treated, or into the tissue of a fistula. The microneedle devices according to the invention are particularly suitable for delivering precise doses of relatively expensive therapeutic fluids, such as stem cells or therapeutic liquids or fluids containing stem cells or other expensive drugs, since the wastage of the therapeutic fluid or liquid is minimised, firstly, due to the fact that the therapeutic fluid or liquid is delivered directly to the location to which it is to be delivered, and secondly, due to the micro nature of the microneedle devices.

Additionally, even although a physician or other person administering the therapeutic fluid or liquid does not have visual access with the site in the lumen, vessel, cavity, organ, muscular organ or fistula at which the therapeutic fluid is being injected into the tissue, the wall or the musculature of the lumen, vessel, cavity, organ or muscular organ, or into the tissue of a fistula, the therapeutic fluid can be delivered with precision at precise known locations.

Another advantage of the microneedle devices according to the invention is that they are particularly suitable for delivering relatively viscous therapeutic substances due to the short length of the microneedles.

A further advantage of the invention is that the microneedle devices according to the invention may be used in any fistula or other lumen, vessel, cavity, organ or muscular organ which is accessible to an endoscope or other such delivery device.

The microneedle devices according to the invention are also suitable for delivering therapeutic substances to organs in the human or animal body, and in particular to muscular organs, such as sphincters, where the delivery devices according to the invention are suitable for the delivery of microbeads and other bulking agents and substances to submucosal surfaces of sphincters and other muscular organs. By injecting a sphincter submucosally with microbeads or other bulking agents or substances, the bore extending through the sphincter can be reduced, thereby improving the sealing function of the sphincter when in the closed state. For example, the microneedle devices according to the invention may be used for injecting the lower oesophageal sphincter submucosally with microbeads or other bulking agents or substances in the treatment of gastroesophageal reflux disease.

The invention will be more clearly understood from the following description of some preferred embodiments thereof which are given by way of example only with reference to the accompanying drawings, which are not to scale, and in which:
Fig. 1 is a cross-sectional side elevational view of a microneedle device according to the invention, but not in its normal use state,
Fig. 2 is a side elevational view of a portion of the microneedle device of Fig. 1 in one state of use,
Fig. 3 is another side elevational view of portions of the microneedle device of Fig. 1 in a different state of use to that of Fig. 2,
Fig. 4 is a transverse cross-sectional end elevational view of the portion of Fig. 2 of the microneedle device of Fig. 1 on the line IV-IV of Fig. 2 in the state of Fig. 2,
Fig. 5 is a transverse cross-sectional end elevational view of the portion of Fig. 3 of the microneedle device of Fig. 1 on the line V-V of Fig. 3 in the state of Fig. 3,
Fig. 6 is a side elevational view of another part of the microneedle device of Fig. 1,
Fig. 7 is a cross-sectional side elevational view of a detail of the portion of Fig. 6 of the microneedle device of Fig. 1,
Fig. 8 is a side elevational view of the microneedle device of Fig. 1 in use,
Fig. 9 is a view similar to Fig. 1 of a microneedle device according to another embodiment of the invention, which is not in its normal use state,
Fig. 10 is a cross-sectional side elevational view of a detail of a microneedle device according to another embodiment of the invention,
Fig. 11 is a view similar to Fig. 10 of a portion of another microneedle device according to a further embodiment of the invention,
Fig. 12 is a side elevational view of a detail of a portion of a microneedle device according to another embodiment of the invention,
Figs. 13 to 18 are side elevational views of a microneedle device according to a further embodiment of the invention in various stages of use,
Fig. 19 is a cross-sectional side elevational view of a portion of a microneedle device according to another embodiment of the invention,
Fig. 20 is a cross-sectional end elevational view of a portion of the microneedle device of Fig. 19 on the line XX-XX of Fig. 19,
Fig. 21 is a cross-sectional side elevational view of a detail of a microneedle device according to another embodiment of the invention,
Fig. 22 is a cross-sectional end elevational view of the detail of Fig. 21 on the line XXII-XXII of Fig. 21 of the microneedle device thereof,
Fig. 23 is a cross-sectional side elevational view of the portion of Fig. 21 on the line XXIII-XXIII of Fig. 22, of the microneedle device thereof,
Fig. 24 is a cross-sectional side elevational view of a detail of another microneedle device according to another embodiment of the invention, and
Fig. 25 is an end elevational view of the portion of Fig. 24 on the line XXV-XXV of Fig. 24 of the microneedle device thereof.

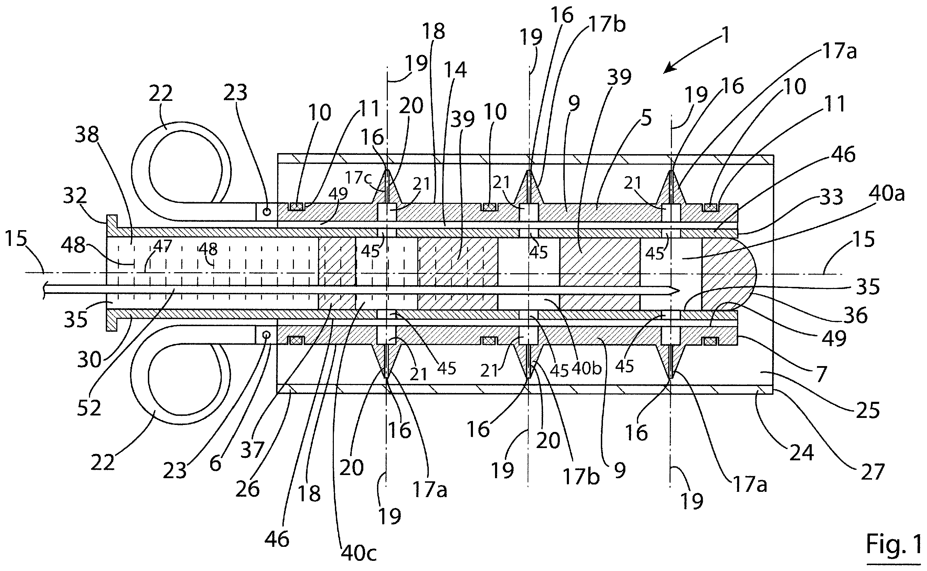

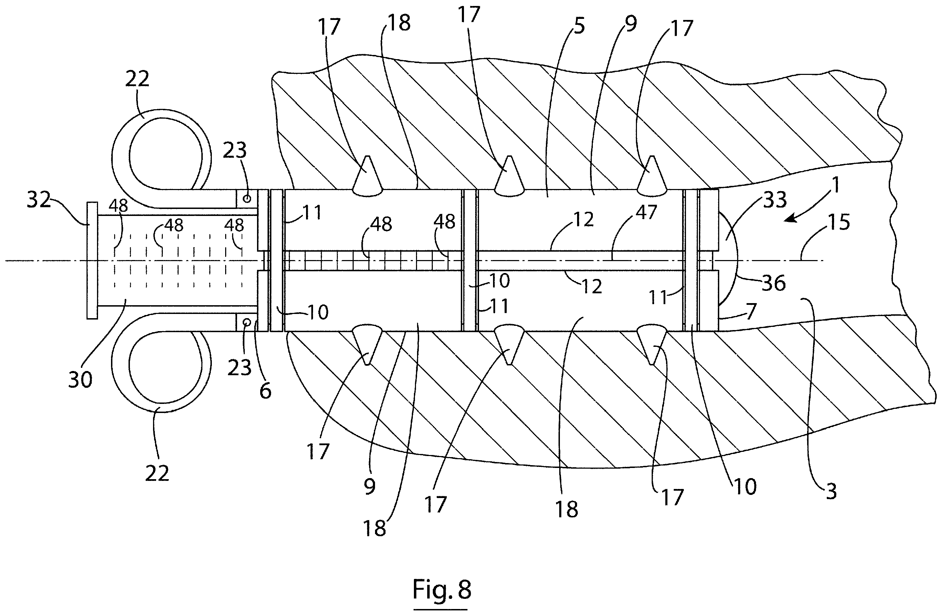

Referring to the drawings and initially to Figs. 1 to 8 thereof, there is illustrated a microneedle device according to the invention indicated generally by the reference numeral 1, which is particularly suitable for use in a method for the treatment of a fistula, and in particular, for use in a method for the treatment of an anal fistula, and in particular, for use in a method for the treatment of a fistula with a therapeutic fluid, for example, a therapeutic fluid comprising stem cells. In Fig. 8 the microneedle device 1 is illustrated located in an anal fistula indicated generally by the reference numeral 3, and its use will be described below. However, it will be appreciated that the microneedle device 1 may be used for the treatment of a site in any lumen, vessel, cavity, organ or muscular organ with a therapeutic fluid, liquid or substance, and in the case of a muscular organ, for example, a sphincter, such as the lower oesophageal sphincter, the microneedle device 1 may be used for injecting microbeads or other bulking agents or substances submucosally into the sphincter for improving the sealing function of the sphincter in the treatment of gastroesophageal reflux disease. It will of course by appreciated that the size, shape and configuration of the various components of the microneedle device according to the invention will be determined largely by the use to which the microneedle device 1 is to be put, and this will be understood by those skilled in the art.

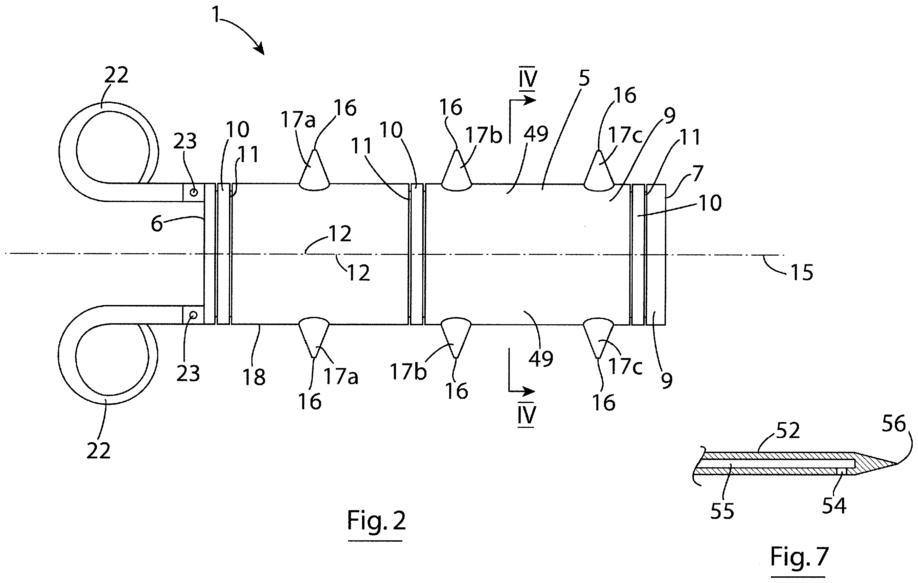

The microneedle device 1 comprises an elongated carrier element 5 extending between a proximal end 6 and a distal end 7. The carrier element 5 is formed by a pair of elongated shells 9 extending between the proximal end 6 and the distal end 7. The shells 9 are of substantially semi-circular transverse cross-section and terminate in longitudinally extending side edges 12. The shells 9 are resiliently urged together into a rest state illustrated in Fig. 2 by a resilient urging means. In this embodiment of the invention the resilient urging means comprises a plurality of bands 10 of a resilient material which are located at longitudinally spaced apart intervals along the shells 9. Annular grooves 11 extending around the shells 9 engage and locate the resilient bands 10 spaced apart along the shells 9. In this embodiment of the invention the resilient bands 10 are of an elastic material, for example, silicone, and three bands 10 are provided, one towards the proximal end 6, the other towards the distal end 7, and a third band 10 substantially midway between the proximal and distal ends 6 and 7. However, it will be readily apparent to those skilled in the art that any suitable number of bands 10 may be provided for resiliently urging the shells 9 together into the rest state. The number of bands 10 will in general be dependent on the overall length of the shells 9. The shells 9 when urged together into the rest state by the bands 10 with the longitudinal side edges 12 thereof abutting each other, form a cylinder defining a main bore 14 of substantially circular transverse cross-section extending longitudinally, and in this embodiment of the invention substantially centrally therethrough from the proximal end 6 to the distal end 7. Additionally, the shells 9 when urged together into the rest state by the bands 10, also define a longitudinally extending main axis 15 which in this embodiment of the invention extends centrally therethrough.

A plurality of microneedles 17 extend radially outwardly from an outer surface 18 of each of the shells 9 on respective opposite sides thereof and terminate in respective tips 16. In this embodiment of the invention six microneedles 17 are provided in pairs spaced apart longitudinally along the outer semicylindrical surfaces 18 of the respective shells 9. The microneedles 17 of each pair thereof are spaced apart circumferentially around the shells 9 at 180° to each other. Each microneedle 17 defines a needle axis 19, which in this embodiment of the invention extends centrally through the microneedle 17, and which extends radially and transversely from the main axis 15. The microneedles 17 of each shell 9 are arranged on the shell 9 with the needle axes 19 of the microneedles 17 aligned axially along the corresponding shell 9. A fluid accommodating bore 20 extends through each microneedle 17 and in this embodiment of the invention communicates with the main bore 14 through a corresponding fluid accommodating inlet port 21 formed in the corresponding one of the shells 9 of the carrier element 5. However, it will be appreciated that the fluid accommodating bore 20 of each microneedle 17 could terminate directly in the main bore 14 of the carrier element 5.

In this embodiment of the invention the microneedles 17 are integrally formed with the corresponding shells 9 and the shells 9 and the microneedle 17 are formed by moulding from polycarbonate material, although any other suitable material may be used. In this embodiment of the invention it is desired to deliver the therapeutic fluid into the tissue adjacent the fistula 3 to a depth of 1mm to 2mm, and accordingly each microneedle 17 is of length along the needle axis 19 thereof from the outer surface 18 of the corresponding shell 9 to the tip 16 of the microneedle 17 of approximately 2mm. It will be understood that the length of each microneedle 17 from the outer surface 18 of the corresponding shell 9 will be dependent on the depth below the surface of the tissue at which the therapeutic fluid is to be delivered.

Finger grip elements 22 are releasably coupled by quick release couplers 23 to the respective shells 9 for facilitating urging the carrier element 5 into the fistula 3 as will be described below. Such quick release couplers as the quick release couplers 23 will be well known to those skilled in the art.

A shielding means, in this embodiment of the invention a tubular shield 24, is provided for shielding the microneedles 17 as the carrier element 5 is being urged into the fistula 3 in order to avoid tearing of the tissue adjacent the fistula by the microneedles 17 as the carrier element 5 is being urged into and through the fistula. The tubular shield 24 is of circular transverse cross-section, and in this embodiment of the invention is of length just greater than the length of the carrier element 5 formed by the shells 9. The tubular shield 24 defines a carrier element accommodating bore 25 extending therethrough from a proximal end 26 to a distal end 27 thereof. The diameter of the carrier element accommodating bore 25 is of diameter just greater than the maximum diameter of the carrier element defined by the tips 16 of the microneedles 17 when the shells 9 are in the rest state with the longitudinal side edges 12 thereof abutting each other. The bore 25 extending through the tubular shell 24 is configured to accommodate the carrier element 5 with the microneedles 17 in the rest state during simultaneous insertion of the tubular shield into the fistula 3, or by sequential insertion of the tubular shield 24 and the carrier element 5 into the fistula 3, with the tubular shield 24 being initially inserted into the fistula 3. The tubular shield 24 is configured for withdrawal from the fistula 3 once the carrier element 5 has been located in the fistula at the site to be treated. Withdrawal of the tubular shield 24 from the fistula 3 is facilitated by removing the finger grip elements 22 from the shells 9 while the tubular shield 24 is being withdrawn from the fistula 3.

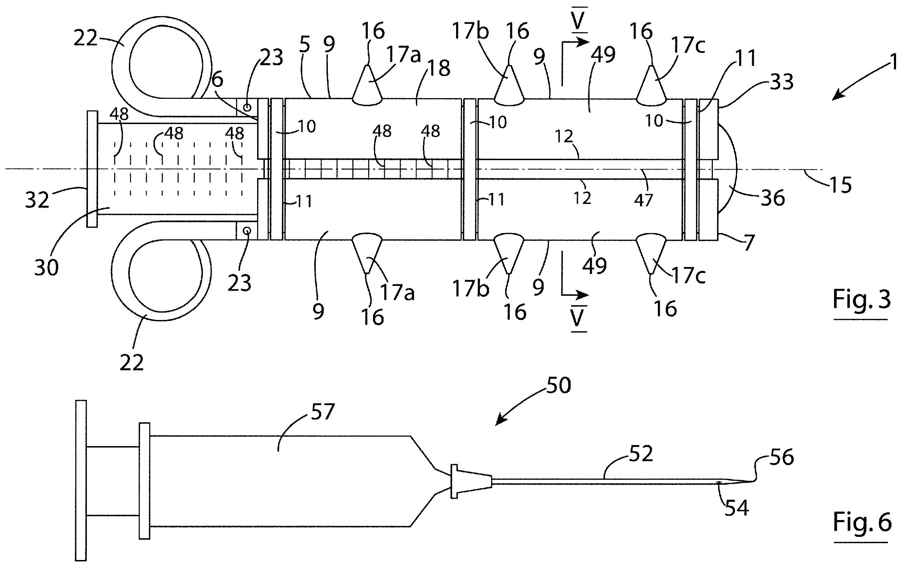

An operating means is provided for urging the shells 9 and in turn the microneedles 17 radially outwardly relative to the main axis 15 from the rest state to an engagement state, which is illustrated in Figs. 3 and 8, for in turn urging the microneedles 17 into the tissue within the fistula 3. In this embodiment of the invention the operating means comprises an elongated tubular operating member 30 of substantially circular external transverse cross-section extending between a proximal end 32 and a distal end 33, and having a bore 35 of circular transverse cross-section extending therethrough from the proximal end 32 to the distal end 33. In this embodiment of the invention the bore 35 extends centrally through the operating member 30. The external diameter of the operating member 30 is greater than the diameter of the main bore 14 extending through the carrier element 5 defined by the shells 9 when the shells 9 are in the rest state, so that when the tubular shield 24 has been withdrawn from the fistula, by urging the operating member 30 into the main bore 14 of the carrier element 5, the action of the operating member 30 on the shells 9 urges the shells 9 radially outwardly against the resilient urging force of the bands 10. Thereby the microneedles 17 are urged transversely and radially outwardly relative to the main axis 15 of the carrier element 5 from the rest state to the engagement state, and are urged into the tissue adjacent the fistula 3. In this embodiment of the invention since it is desirable that the therapeutic fluid is to be injected at a depth of approximately 1mm to 2mm from the fistula 3, the relationship between the external diameter of the operating member 30 and the diameter of the main bore 14 of the carrier element 5 is such that the shells 9 are urged apart from each other by the operating member 30 a distance of approximately 4mm. This is sufficient for urging the microneedles 17 into the adjacent tissue of the fistula 3 to a depth of approximately 2mm. However, the external diameter of the operating member could be such as to urge the shells 9 apart a radial distance greater than 4mm, since the depth of penetration of the microneedles 17 into the tissue adjacent the fistula 3 will be no greater than the length of the microneedles 17, which in this embodiment of the invention is approximately 2mm.

The bore 35 in the operating member 30 is sealably closed at the distal end 33 thereof by a distal end sealing means comprising a distal end plug 36 of circular transverse cross-section sealably engaged in the bore 35 adjacent the distal end thereof. The bore 35 of the operating member 30 is also sealably closed towards the proximal end 32 thereof by a proximal sealing means comprising a proximal end plug 37 of circular transverse cross-section sealably engaged in the bore 35 towards the proximal end 32 thereof.

A communicating means is provided for communicating the microneedles with a source of the therapeutic fluid. In this embodiment of the invention the communicating means comprises three longitudinally spaced apart main chambers 40 formed in the bore 35 of the operating member 30 between the distal and proximal end plugs 36 and 37, respectively. A pair of spaced apart intermediate sealing means, namely, intermediate sealing plugs 39 of circular transverse cross-section sealably engaged in the bore 35 spaced apart from the corresponding ones of the distal and proximal end plugs 36 and 37 form with the proximal and distal end plugs 36 and 37 the main chambers 40 in the operating member 30.

The proximal and distal end plugs 36 and 37 and the intermediate end plugs 39 form a first sealing means for sealably isolating the main chambers 40 from each other. The main chambers 40 in this embodiment of the invention are configured as will be described below to accommodate the therapeutic fluid therethrough from the fluid source to the fluid accommodating bores 20 of the microneedles 17. The main chambers 40 in the operating member 30 are alignable with corresponding pairs of the microneedles 17 and are communicable with the fluid accommodating bores 20 of the corresponding pairs of microneedles 17, as will be described in more detail below.

A fluid delivery means, namely, an elongated fluid delivery needle 52 is provided for delivering the therapeutic fluid from the source to the main chambers 40. In this embodiment of the invention the therapeutic fluid is located in a syringe 51 of a needle and syringe assembly 50, which also comprises the delivery needle 52, for delivery to the main chamber 40 through the delivery needle 52 and in turn through the microneedles 17. The needle and syringe assembly 50 and the delivery needle 52 will be described in more detail below.

The bore 35 of the operating member 30 forms a needle accommodating duct 38 for accommodating the delivery needle 52 as will be described below sequentially into the main chambers 40. The proximal end plug 37 and the intermediate end plugs 39 comprise a resilient, puncturable, self-resealing material for sealably accommodating the delivery needle 52 sequentially into the main chambers 40, and on removal of the delivery needle 52, the proximal end plug 37 and the intermediate end plugs 39 reseal themselves.

A pair of communicating ports 45 extending through the tubular operating member 30 from each main chamber 40 communicate the main chambers 40 with the fluid accommodating bores 20 of the respective corresponding pairs of microneedles 17 through the corresponding inlet ports 21 of the microneedles 17. The communicating ports 45 are of cross-sectional area substantially similar to the cross-sectional area of the inlet ports 21 in the carrier element 5, and are simultaneously alignable with the corresponding inlet ports 21 for transferring a therapeutic fluid from each main chamber 40 to the corresponding pair of microneedles 17, when the operating member 30 is located in the main bore 14 of the carrier element 5 and fully aligned with the carrier element 5, both circumferentially and longitudinally, as will be described below.

An alignment means comprising a circumferential alignment means and a longitudinal alignment means are provided for aligning the operating member 30 both circumferentially and longitudinally with the carrier element, so that the communicating ports 45 of the operating member 30 are aligned with the corresponding inlet ports 21 of the carrier element 5. The circumferential alignment means comprises a longitudinally extending alignment mark, in this embodiment of the invention provided by an elongated longitudinal alignment graduation 47. The longitudinal alignment graduation 47 is provided on the operating member 30, so that when the longitudinal alignment graduation 47 is located midway between two of the adjacent butting edges 12 of the shells 9, the operating member 30 is circumferentially aligned with the carrier element 5, and the communicating ports 45 of the operating member 30 are aligned circumferentially with the corresponding inlet ports 21 of the shells 9.

The longitudinal alignment means for longitudinally aligning the operating member 30 with the carrier element 5 comprises a plurality of longitudinally spaced apart alignment marks, which in this embodiment of the invention are provided by transverse alignment graduations 48 provided on the operating member 30 towards the proximal end 32 thereof. The transverse alignment graduations 48 are provided for longitudinally aligning the operating member 30 with the proximal end 6 of the carrier element 5 for in turn longitudinally aligning the communicating ports 45 of the operating member 30 with the corresponding inlet ports 21 in the shells 9 of the carrier element 5.

In this embodiment of the invention the tubular operating member 30 is of non-deformable transverse cross-section, and may comprise polycarbonate or ABS material, and is of sufficient strength to be non-deformable in transverse cross-section, so as not to deform under the resilient action of the bands 10 as the operating member 30 is being urged through the main bore 14 of the carrier element 5. Additionally, the shells 9 are of sufficient resilience and flexibility, so that when the operating member 30 is urged through the main bore 14 of the carrier element 5 and the shells 9 are urged outwardly against the resilient action of the bands 10, the inner surface 49 of each of the shells 9 tightly and sealably abut the outer surface 46 of the operating member 30 adjacent the inlet ports 21 of the shells 9 and the communicating ports 45 of the operating member 30 for providing a fluid-tight seal between the operating member 30 and the shells 9 around the inlet ports 21 and the corresponding communicating ports 45 when the operating member 30 is longitudinally and circumferentially aligned with the carrier element 5.

Turning now to the needle and syringe assembly 50, the needle and syringe assembly 50 comprises the syringe 51 and the delivery needle 52 secured to the syringe 51 and communicating therewith. The syringe 51 in this embodiment of the invention forms the source of the therapeutic fluid, although the therapeutic fluid could be delivered from any suitable remote source, for example, from a remote source by a rotary incrementally operated pump. The delivery needle 52 comprises a Huber needle, although, any other suitable needle may be used, but typically, the delivery needle 52 will be of the type which comprises a side outlet port 54 communicating with a bore 55 extending through the needle to the side port 54, see Figs. 6 and 7. A distal end 56 of the delivery needle 52 is closed and pointed for piercing and extending through the proximal end plug 37 and one or more of the intermediate plugs 39 until the distal end 56 of the delivery needle 52, and the side port 54 thereof are located in the selected one of the main chambers 40. In Fig. 1 the distal end 56 and the side outlet port 54 of the delivery needle 52 are illustrated in the distal most main chamber 40a of the operating member 30. Once the distal end 56 and the side port 54 of the delivery needle 52 are located in the selected main chamber 40, the therapeutic fluid is urged by the syringe 51 and the delivery needle 52 into and through the selected main chamber 40, and in turn through the corresponding communicating ports 45 of the operating member 30, the inlet ports 21 and the fluid accommodating bores 20 of the corresponding pair of microneedles 17, and in turn into the tissue of the subject within the fistula 3.

While in Fig. 1 the carrier element 5 is illustrated located within the carrier element accommodating bore 25 of the tubular shield 24 with the operating member 30 located within the main bore 14 of the carrier element 5, in practice this is not possible since the diameter of the carrier element accommodating bore 25 of the tubular shield 24 is just sufficient to accommodate the carrier element 5 with the microneedles 17 and the shells 9 in the rest state therein. Fig. 1 has been provided solely for the purpose of illustrating all the components of the microneedle device 1 together.

In use, with the tubular operating member 30 removed entirely from the main bore 14 extending through the carrier element 5, the carrier element 5 with the microneedles 17 in the rest state is located in the carrier element accommodating bore 25 of the tubular shield 24 with the microneedles 17 fully shielded by the tubular shield 24. With the finger grip elements 22 secured to the shells 9 of the carrier element 5, the assembly of the carrier element 5 within the tubular shield 24 is urged from the anus into the fistula 3. The assembly of the tubular shield 24 and the carrier element 5 is urged through the fistula 3 until the assembly is at a desired location in the fistula, namely, the site to be treated in the fistula 3. The finger grip elements 22 are then detached from the shells 9, and the tubular shield 24 is removed from the fistula 3, leaving the carrier element 5 in the desired location in the fistula 3. The finger grip elements 22 are reattached to the shells 9 for holding the carrier element 5 in position in the fistula 3.

The tubular operating member 30 is then urged into the main bore 14 of the carrier element 5 for urging the shells 9 transversely and radially outwardly relative to the main axis 15 from the rest state to the engagement state. During urging of the operating member 30 into the main bore 14 of the carrier element 5 the longitudinal alignment graduation 47 is maintained in alignment centrally between the adjacent abutting edges 12 of the shells 9 in order to circumferentially align the communicating ports 45 in the operating member 30 with the corresponding inlet ports 21 in the shells 9. The operating member 30 is urged into the main bore 14 of the carrier element 5 until the appropriate one of the transverse alignment graduations 48 is aligned with the proximal end 6 of the carrier element 5. In general, it is envisaged that the operating member 30 will be inserted into the main bore 14 of the carrier element 5, so that the communicating ports 45 of the distal most main chamber 40a are longitudinally aligned with the inlet ports 21 of the distal most microneedles 17a. Although, in certain uses of the microneedle device 1, it is envisaged that the distal most main chamber 40a need not necessarily be aligned with the distal most microneedles 17a. The distal most main chamber 40a may be aligned with any of the microneedles 17a to 17c.

Once the operating member 30 is located in the main bore 14 of the carrier element 5 with the communicating ports 45 of the operating member 30 aligned with the inlet ports 21 of the corresponding microneedles 17, the microneedles 17 should be fully engaged in the tissue adjacent the fistula 3 to a depth of between 1mm and 2mm from the fistula 3. The therapeutic fluid is then injected through the microneedles 17 by the needle and syringe assembly 50 into the tissue of the subject adjacent the fistula 3. With the syringe 51 of the syringe assembly charged with the therapeutic fluid, the delivery needle 52 of the needle and syringe assembly 50 is urged through the proximal end plug 37 and through the intermediate plugs 39 until the distal end 56 and the side outlet port 54 of the delivery needle 52 are located in the one of the main chambers 40 which is aligned with the microneedles 17 through which the therapeutic fluid is to be injected into the tissue adjacent the fistula 3. Typically, initially the delivery needle 52 is urged into the proximal most main chamber 40, namely, the main chamber 40c. With the distal end 56 and the side outlet port 54 of the delivery needle 52 located in the proximal most main chamber 40c, the therapeutic fluid is injected through the delivery needle 52 into the proximal most main chamber 40c and in turn through the communicating ports 45 and the inlet ports 21 and in turn through the fluid accommodating bores 20 of the proximal most microneedles 17c into the adjacent tissue of the subject.

On completion of that injection, the syringe 51 is detached from the delivery needle 52 and recharged with the next dose of the therapeutic fluid. The syringe 51 is then reassembled to the delivery needle 52 and the delivery needle 52 is urged into the next main chamber 40, namely, the main chamber 40b, and the injection of the therapeutic fluid is repeated. This process is again repeated for injecting the therapeutic fluid through the delivery needle 52 into the distal most main chamber 40a and in turn through the distal most microneedles 17a.

On completion of the treatment of the subject with the therapeutic fluid, the delivery needle 52 is removed from the operating member 30, and the operating member 30 is then removed from the main bore 14 of the carrier element 5. The finger grip elements 22 are detached from the shells 9, and the tubular shield 24 is urged into the fistula 3 over the carrier element 5 until the carrier element 5 and the microneedles 17 are fully within the carrier element accommodating bore 25 of the tubular shield 24. With the carrier element 5 fully located within the tubular shield 24, the finger grip elements 22 are again secured to the shells 9. With the tubular shield 24 shielding the microneedles 17, the assembly of the tubular shield 24 and the carrier element 5 may be urged further into the fistula 3 in order to treat tissue deeper into the fistula, or the assembly of the tubular shield 24 and the carrier element 5 may be withdrawn from the fistula 3 and in turn withdrawn through the anus.

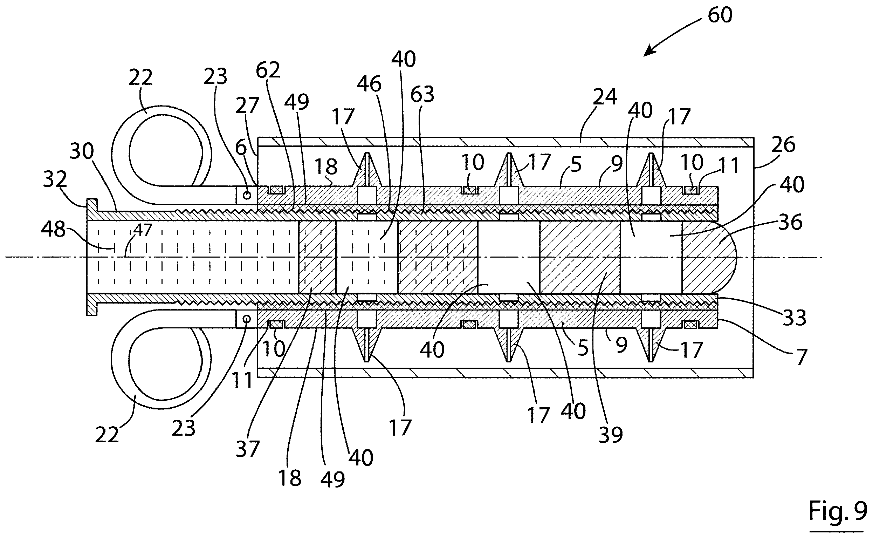

Referring now to Fig 9 there is illustrated a microneedle device according to another embodiment of the invention indicated generally by the reference numeral 60. The microneedle device 60 is also suitable for use in a method for the treatment of a fistula with a therapeutic fluid, for example, for use in the treatment of tissue adjacent a fistula with a therapeutic fluid comprising stem cells. The microneedle device 60 is substantially similar to the microneedle device 1, and similar components are identified by the same reference numerals.

The main difference between the microneedle device 60 and the microneedle device 1 is that the main bore 14 defined by the shells 9 is internally threaded with screw threads 62 adjacent the proximal end 6 thereof which are engageable with corresponding external screw threads 63 provided along the outer surface 46 of the operating member 30 from the proximal end 32 to the distal end 33 thereof. The external screw threads 63 on the operating member 30 co-operate with the internal threads 62 in the main bore 14 of the carrier element 5, so that by appropriately rotating the operating member 30 in the main bore 14 of the carrier element 5, the operating member 30 advances through the main bore 14 of the carrier element 5 for in turn urging the shells 9 transversely and radially outwardly from the main axis 15 from the rest state to the engagement state. Reverse rotation of the operating member 30 in the main bore 14 results in withdrawal of the operating member 30 from the carrier element 5.

The longitudinal alignment graduation 47 and the transverse alignment graduations 48 are provided for circumferentially and longitudinally aligning the operating member 30 with the carrier element 5 as already described with reference to the microneedle device 1.

Otherwise, the microneedle device 60 and its use is similar to that of the microneedle device 1. However, although the microneedle device 60 is illustrated in Fig. 9 with the carrier element 5 located within the carrier element accommodating bore 25 of the tubular shield 24, and with the operating member 30 located within the main bore 14 of the carrier element 5, as already discussed with reference to Fig. 1, in practice the diameter of the carrier element accommodating bore 25 of the tubular shield 24 is such that the carrier element can only be accommodated within the carrier element accommodating bore 25 of the tubular shield 24 when the operating member 30 is completely withdrawn from the main bore 14 of the carrier element 5 and the microneedles 17 are in the rest state.

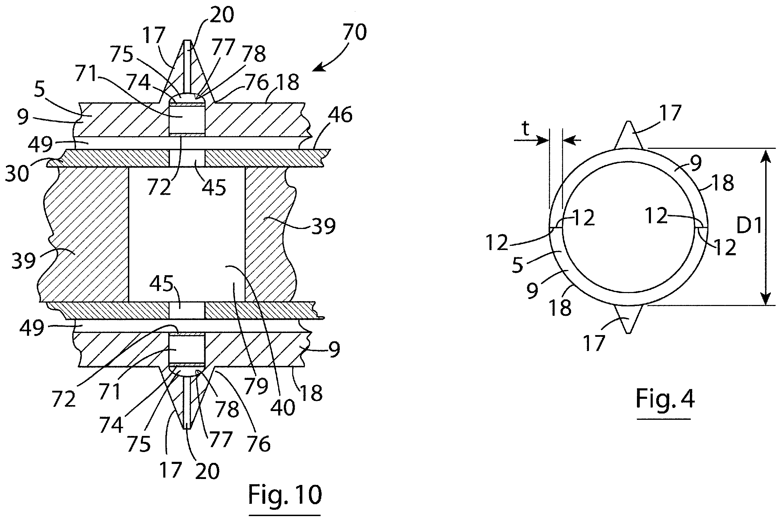

Referring now to Fig. 10 there is illustrated a portion 70 a microneedle device according to another embodiment of the invention. This microneedle device, the portion 70 of which is illustrated, is substantially similar to the microneedle device 1, and similar components are identified by the same reference numerals. The main difference between this microneedle device 70 and the microneedle device 1 lies in the shells 9 of the carrier element 5. In this embodiment of the invention main chambers 71 for storing the therapeutic fluid are located in the carrier element 5, and in this case are located in the shells 9 of the carrier element 5. Each main chamber 71 in this embodiment of the invention is configured to store the therapeutic fluid, and is of size sufficient to contain a single dose of the therapeutic fluid, which may or may not comprise stem cells.

Each main chamber 71 is of cylindrical shape and is isolated from the main bore 14 of the carrier element 5 by an urging means for urging the therapeutic fluid from the main chamber 71 and through the fluid accommodating bore 20 of the corresponding microneedle 17. In this embodiment of the invention each urging means comprises a resilient and flexible, elastically expandable, main membrane 72, which is sealably secured to the shell 9 in order to sealably isolate the main chamber 71 from the main bore 14 extending through the carrier element 5. In this embodiment of the invention the main membrane 72 is of circular shape.

Each main chamber 71 is also isolated from the fluid accommodating bore 20 extending through the corresponding microneedle 17 by a resilient and flexible, elastically expandable, rupturable secondary membrane 74 also of circular shape. The fluid accommodating bore 20 of each microneedle 17 terminates in an intermediate chamber 75 located towards the base 76 of the corresponding microneedle 17 between the main chamber 71 and the fluid accommodating bore 20. Each secondary membrane 74 is located between the intermediate chamber 75 and the corresponding main chamber 71 for sealably isolating the main chamber 71 from the intermediate chamber 75, for in turn retaining the therapeutic fluid in the main chamber 71. A piercing member 77 extends from each microneedle 17 into the intermediate chamber 75 thereof and terminates in a piercing point 78 within the intermediate chamber 75 spaced apart from the secondary membrane 74. The secondary membrane 74 is stretched taut and the piercing member 77 is located in the intermediate chamber 75 so that when the secondary membrane 74 is urged into the intermediate chamber 75, the piercing point 78 of the piercing member 77 ruptures the secondary membrane 74 in order to permit the dose of the therapeutic fluid in the main chamber 71 to be urged through the fluid accommodating bore 20 in the corresponding microneedles 17.

In this embodiment of the invention the tubular operating member 30 is substantially similar to the tubular operating member 30 of the microneedle device 1, with the exception that the chambers 40, which in the operating member 30 of the microneedle device 1 act as main chambers, in this embodiment of the invention, the chambers in the operating member 30 act as secondary chambers which are indicated by the reference numeral 79, and are provided for accommodating a drive medium, which in this case comprises pressurised air or other gas, for in turn urging the main membranes 72 into the corresponding ones of the main chambers 71 in the shells 9, for in turn urging the therapeutic fluid from the main chambers 71 through the fluid accommodating bores 20 in the corresponding microneedles 17. The communicating ports 45 in the operating member 30 from the secondary chambers 79 are alignable with the main membranes 72 of the corresponding main chambers 71, in a similar manner as the communicating ports 45 of the operating member 30 of the microneedle device 1 described with reference to Figs. 1 to 8 are alignable with the inlet ports 21 of the carrier element 5 of the microneedle device 1. In this embodiment of the invention the communicating ports 45 deliver the pressurised air from the secondary chambers 79 to the corresponding main membranes 72 for urging the main membranes 72 into the corresponding main chambers 71.

The pressurised air supply is delivered by a drive medium delivery means sequentially or simultaneously, but generally sequentially into the respective secondary chambers 79 of the operating member 30 when the operating member 30 is located fully in the main bore 14 of the carrier element 5 and aligned therewith. In this embodiment of the invention the drive medium delivery means comprises a drive medium delivery needle which is similar to the delivery needle 52 of the needle and syringe assembly 50. In this embodiment of the invention the drive medium delivery needle is also identified by the reference numeral 52, the corresponding syringe and the corresponding syringe assembly are also identified by the reference numeral 51 and 50, respectfully. The delivery needle 52 is sequentially entered into the secondary chambers 79 in a similar manner as described with reference to the microneedle device 1, and air in the syringe 51 is urged into the secondary chamber 79 in which the side outlet port 54 of the delivery needle 52 is located, for in turn urging the main membranes 72 into the main chambers 71 of the microneedles 17 which correspond with the secondary chamber 79 into which the pressurised air is being delivered by the syringe 51.

In use, in the treatment of an anal fistula, when the carrier element 5 has been located in the fistula with the tubular shield having been withdrawn from the fistula, the operating member 30 is urged into the main bore 14 of the carrier element 5. When the operating member 30 is fully located in the main bore 14 of the carrier element 5 and aligned therewith with the communicating ports 45 aligned with the corresponding main membranes 72 of the main chambers 71 in the shells 9 of the carrier element 5, the delivery needle 52 attached to the syringe 51 of the needle and syringe assembly 50 is urged through the proximal end plug 37 into the proximal most secondary chamber 79. With the distal tip 56 of the delivery needle 52 and the side outlet port 54 located in the proximal secondary chamber 79, air is delivered into the proximal secondary chamber 79 from the syringe 51. The pressurised air being delivered into the secondary chamber 79 is in turn urged through the communicating ports 45 and in turn acts on the corresponding main membranes 72 of the corresponding proximal most main chambers 71. The action of the pressurised air on the main membranes 72 urges the main membranes 72 into the corresponding main chambers 71, which in turn raises the pressure of the therapeutic fluid in the main chambers 71, thereby resulting in the secondary membranes 74 being urged against the piercing points 78 of the piercing members 77 for rupturing thereof. Further urging of the main membranes 72 into the corresponding main chambers 71 under the action of the pressurised air results in the therapeutic fluid in the main chambers 71 being discharged through the fluid accommodating bores 20 of the corresponding microneedles 17 and in turn injected into the adjacent tissue of the fistula.

The delivery needle 52 is then sequentially urged into the remaining secondary chambers 79 for in turn delivering pressurised air into the respective secondary chambers 79 for in turn discharging the therapeutic fluid from the corresponding main chambers 71 through the corresponding microneedles 17. On completion of the delivery of the therapeutic fluid from the main chambers 71 into the tissue of the fistula, the carrier element is removed from the fistula in a similar manner to that described with reference to the removal of the carrier element of the microneedle device 1 from the fistula.

Otherwise, the microneedle device 70 and its use is similar to that of the microneedle device 1.

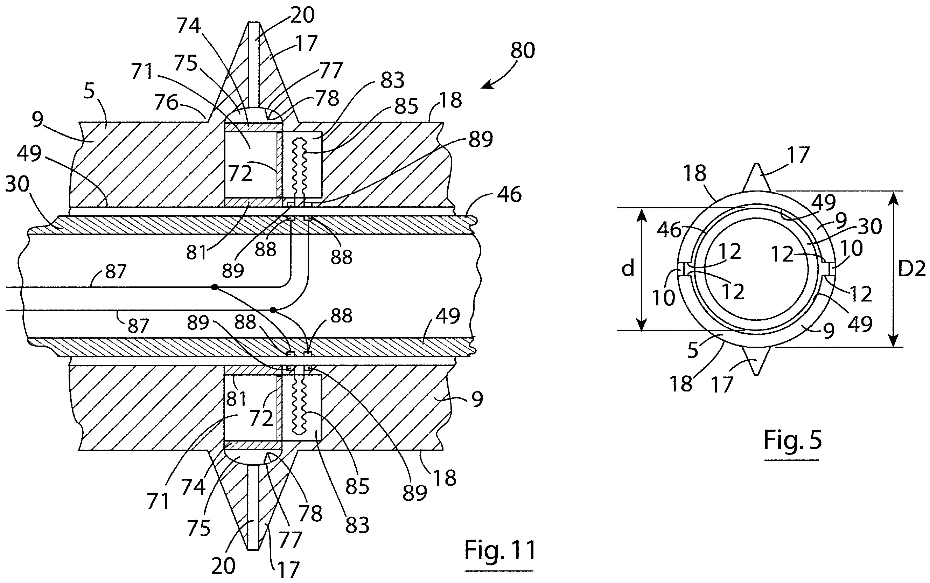

Referring now to Fig. 11 there is illustrated a portion 80 of a microneedle device according to another embodiment of the invention for delivering a therapeutic fluid which may or may not comprise stem cells into tissue adjacent a fistula 3. This microneedle device is substantially similar to the microneedle device 70, and in turn is substantially similar to the microneedle device 1 and similar components are identified by the same reference numeral as those of the microneedle devices 1 and 70 as appropriate. In this embodiment of the invention the shells 9 are substantially similar to the shells 9 of the microneedle device 70 and each defines cylindrical main chambers 71, each of which is configured to store the therapeutic fluid, and is pre-charged with a dose of the therapeutic fluid. In this embodiment of the invention the main chambers 71 are sealably isolated from the main bore 14 of the carrier element 5 by a rigid wall 81 formed integrally with the corresponding shell 9. In this embodiment of the invention each main chamber 71 is isolated from the fluid accommodating bore 20 of the corresponding microneedle 17 by a secondary membrane 74 similar to the secondary membrane 74 of the microneedle device 70. Each microneedle 17 is provided with an intermediate chamber 75 in the base 76 thereof with a piercing member 77 extending into the intermediate chamber 75 with a piercing point 78 in the intermediate chamber 75 spaced apart from the second membrane 74 for rupturing thereof, as described with reference to the microneedle device 70.

Each main chamber 71 comprises an urging means for urging the therapeutic fluid from the main chamber 71 through the fluid accommodating bores 20 of the corresponding microneedles 17. Each urging means in this embodiment of the invention comprises a main membrane 72, of similar material to the main membrane 72 of the microneedle device 70. However, in this embodiment of the invention secondary chambers 83 corresponding to the respective main chambers 71 are provided in the shells 9 of the carrier element 5. Each secondary chamber 83 is provided for accommodating a drive medium for urging the main membrane into the corresponding main chamber 71 for urging the therapeutic fluid from the main chamber 71 and through the corresponding microneedle 17. The main membranes 72 are located between the respective main chamber 71 and the corresponding secondary chambers 86, and each main membrane 72 sealably isolates the corresponding main chamber 71 from the corresponding secondary chamber 83. In this embodiment of the invention each secondary chamber 83 is charged with the drive medium, which in this case comprises an expandable medium, which in this embodiment of the invention comprises microbeads (not shown) of the type which expand to a multiple of their size, typically, sixty times their size when subjected to heat.

An activating means, in this embodiment of the invention a heating means, provided in this case by an electrically powered heating element 85 is located in each secondary chamber 83 for heating the microbeads to expand for in turn urging the main membrane 72 into the corresponding main chamber 71, for in turn discharging the therapeutic fluid therefrom through the fluid accommodating bore 20 of the corresponding microneedle 17.

In this embodiment of the invention the operating member 30 may be tubular or solid, and need not be provided with main chambers 40 or secondary chambers 79. However, in this embodiment of the invention the operating member 30 is provided with pairs of electrical contacts 88 on the outer surface 46 thereof, which when the operating member 30 is fully longitudinally and circumferentially aligned with the carrier element 5 the electrical contacts 88 on the outer surface 46 of the operating member 30 are aligned with and engage corresponding pairs of electrical contacts 89 on the inner surface 49 of the shells 9 which are connected to the corresponding heating elements 85 in the secondary chambers 83 in the shells 9. Wires 87 extending from the electrical contacts 88 of the operating member 30 extend through the operating member 30 to an appropriately switched electrical power supply (not shown) for selectively supplying electrical power to the electrical contacts 88 of the operating member 30. Typically, it is envisaged that a common neutral wire would be provided to one of each pair of the electrical contacts 88 of the operating member 30, and separate live wires would be provided to the other ones of the pairs of electrical contacts 88 of the operating member 30 for facilitating operation of the heater elements 85 independently of each other. Thus, when the operating member 30 is fully longitudinally and circumferentially aligned with and within the carrier element 5, electrical power may be supplied sequentially or simultaneously to the heating elements 85 through the electrical contacts 88 and 89 for in turn expanding the microbeads in the secondary chambers 83.

In use, with the carrier element 5 located in the desired location in the fistula 3, on withdrawal of the tubular shield 24 from the fistula 3, the operating member 30 is urged into the main bore 14 of the carrier element 5 for urging the microneedles 17 into the tissue adjacent the fistula 3. The operating member 30 is longitudinally and circumferentially aligned with and within the carrier element 5, so that the electrical contacts 88 on the outer surface 46 of the operating member 30 are aligned with and in electrical engagement with the corresponding electrical contacts 89 of the heating elements 85 in the secondary chambers 83 of the shells 9. Electrical power is then supplied either sequentially or simultaneously to the heating elements 85 for in turn expanding the microbeads to in turn urge the therapeutic fluid from the main chambers 71 through the fluid accommodating bores 20 of the corresponding microneedles 17 into the tissue of the subject adjacent the fistula.

Otherwise, the microneedle device 80 and its use is similar to that of the microneedle device 70.

Alternatively, in the microneedle device 80, instead of providing the electrical power to the heating elements 85 through the electrical contacts 88 on the operating member 30 and the electrical contacts 89 on the inner surfaces 49 of the shells 9, it is envisaged that the heating elements 85 may be connected directly to electrically conductive wires extending through the shells 9 from the electrical heating elements 85 to the proximal ends 6 of the shells 9. Suitable connectors would be provided on the proximal ends 6 of the shells 9 for connecting the heating elements 85 to the switched electrical power supply (not shown).

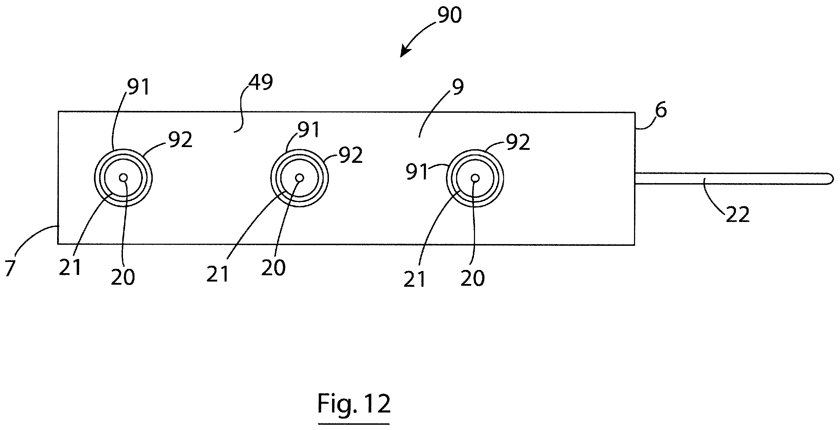

Referring now to Fig. 12 there is illustrated a portion 90 of a microneedle device according to another embodiment of the invention. The portion 90 of the microneedle device illustrated in Fig. 12 comprises one of the shells 9 of the carrier element of the microneedle device according to this embodiment of the invention. The shell 9 of the microneedle device according to this embodiment of the invention is substantially similar to the shell 9 of the microneedle device 1, and similar components are identified by the same reference numerals. The shell 9 of this embodiment of the invention may be used in place of each of the shells 9 of the carrier element 5 of each and anyone of the microneedle devices 1, 60 and 70 already described. In this embodiment of the invention the shell 9 is provided with O-ring seals 91 on the inner surface 49 thereof extending around the respective inlet ports 21 of the shell 9. Each O-ring seal 91 is partially recessed into a circular groove 92 extending around adjacent but slightly spaced apart from the corresponding inlet port 21. The O-ring seals 9 are configured to engage the outer surface 46 of the operating member 30 around the corresponding communicating port 45 of the operating member 30 in order to provide a fluid-tight seal between the operating member 30 and the shells 9 around the communicating port 45 and the inlet port 21. It is envisaged in the case of the microneedle device 70 that the O-ring seals 9 would extend around and be slightly spaced apart from the main membranes 72.

It will be appreciated that in some embodiments of the invention the O-ring seals 91 instead of being provided on the shells 9 may be located in grooves formed on the outer surface 46 of the operating member 30 around the communicating ports 45 thereof. Needless to say any other suitable sealing means besides O-ring seals may be provided. It is also envisaged that pairs of spaced apart O-ring seals may extend circumferentially around the outer surface 46 of the operating member 30 on the opposite sides of each communicating port 45 for sealably engaging the outer surface 46 of the operating member 30 with the inner surface 49 of the shells 9 of the carrier element 5 on respective opposite sides of the communicating ports 45 and the corresponding inlet ports 21.

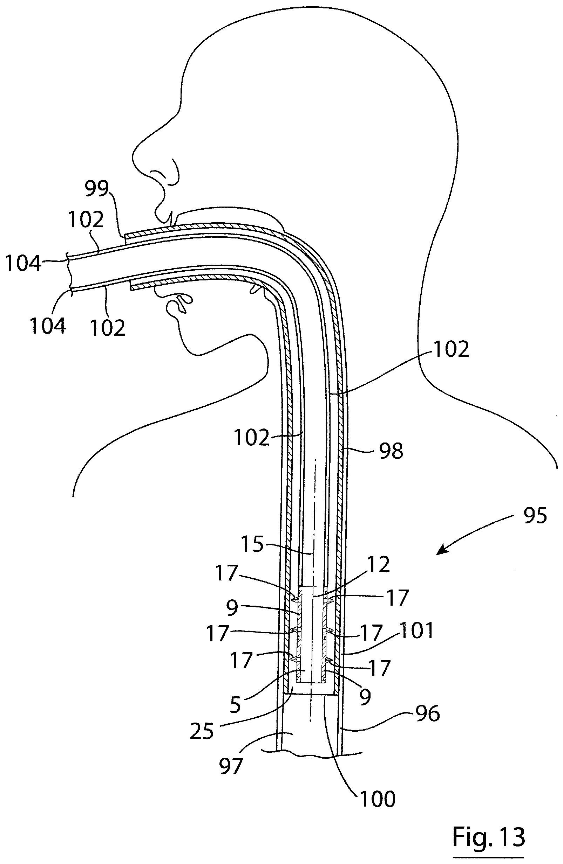

Referring now to Figs. 13 to 18 there is illustrated a microneedle device according to another embodiment of the invention indicated generally by the reference numeral 95. The microneedle device 95 is substantially similar to the microneedle device 1 described with reference to Figs. 1 to 8, and similar components are identified by the same reference numerals. However, in this embodiment of the invention the microneedle device 95 is particularly suitable for use in a method for treating a wall 96 of an oesophagus 97 or other part of the alimentary canal, for example, the duodenum, colon, or other such parts of the alimentary canal, of a human or animal subject with a therapeutic liquid. The therapeutic liquid would typically comprise stem cells. The microneedle device 95 comprises a carrier element 5 which is substantially similar to the carrier element 5 of the microneedle device 1, with the exception that the finger grip elements 92 are omitted. The operating member 30 of the microneedle device 95 is similar to the operating member 30 of the microneedle device 1.

However, in this embodiment of the invention the shielding means for accommodating the carrier element 5 during insertion and removal of the carrier element 5 into and from the oesophagus comprises an elongated tubular shield 98 of transparent material extending between a proximal end 99 and a distal end 100. The length of the tubular shield 98 in this embodiment of the invention is largely determined by the location of the site in the oesophagus or other part of the alimentary canal in which the treatment is to be applied. However, the tubular shield 98 is of sufficient length to extend from the proximal end 99 exteriorly of the subject to the site at which the treatment is to be applied. In this embodiment of the invention the tubular shield 98 is suitable for inserting orally into the oesophagus 97, although in certain cases, the tubular shield 98 may be configured for nasal insertion into the oesophagus. In this embodiment of the invention the carrier element accommodating bore 25 extends longitudinally through the tubular shield 98 from the distal end 100 to the proximal end 99 for accommodating both the carrier element 5 and the operating member 30 therethrough from the proximal end 99 to the distal end 100.

In order to assist in an understanding of the microneedle device 95, the use of the microneedle device 95 in treating a site 101 in the wall 96 of the oesophagus 97 with an injectable therapeutic liquid will now be described.

Initially the carrier element 5 is located in the carrier element accommodating bore 25 adjacent the distal end 100 of the tubular shield 98. Flexible carrier members 102 are secured to the respective shells 9 of the carrier element 5 in place of the finger grip elements 22, and the carrier element 5 is located in the carrier element accommodating bore 25 adjacent to the distal end thereof with the carrier members 102 extending from the respective shells 9 through the carrier element accommodating bore 25 and in turn through the proximal end 99 of the tubular shield 98.

With the carrier element 5 so located in the tubular shield 98 and with the carrier members 102 extending from the tubular shield 98 adjacent to the proximal end 99 thereof, the tubular shield 98 is inserted orally into the oesophagus 97 as illustrated in Fig. 13. The tubular shield 98 is urged through the oesophagus 97 until the distal end 100 of the tubular shield 98, and in turn the carrier element 5 are located adjacent the site 101 to be treated with the therapeutic liquid. An endoscope (not shown) is located in the tubular shield 98 during inserting of the tubular shield 98 in the oesophagus, in order to allow accurate visual positioning of the distal end 100 of the tubular shield 98 and in turn the carrier element 5 in the oesophagus 97 adjacent the site 101 to be treated.

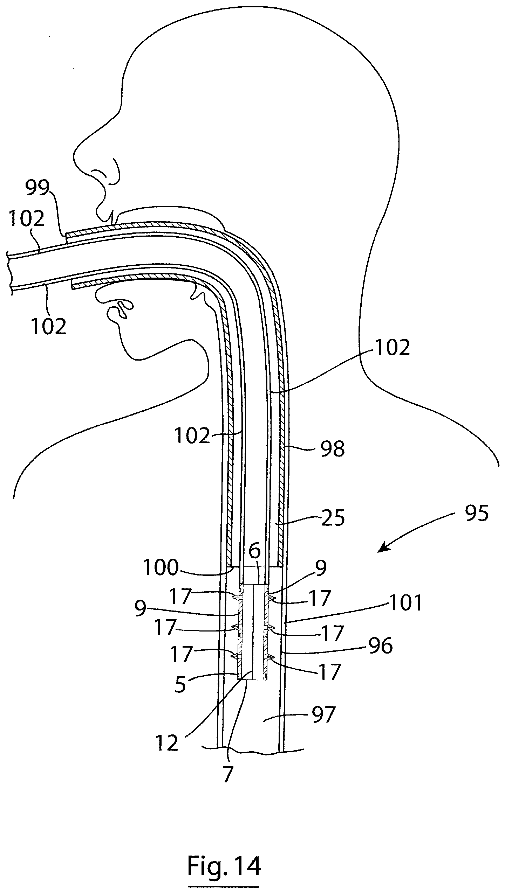

Once the distal end of the tubular shield 98 and in turn the carrier element 5 are accurately located at the site 101 to be treated, the tubular shield 98 is partially withdrawn in order to fully expose the carrier element 5 in the oesophagus 97, see Fig. 14. At this stage the carrier element 5 is maintained positioned at the site 101 by the carrier members 102, the proximal ends 104 thereof may be secured by tape or other means to the subject, for example, adjacent the nose or the cheeks of the subject.

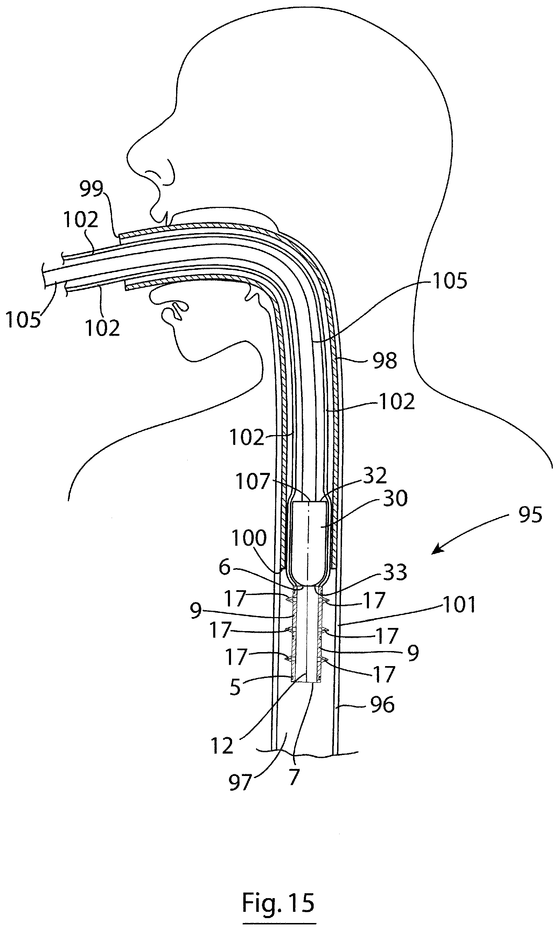

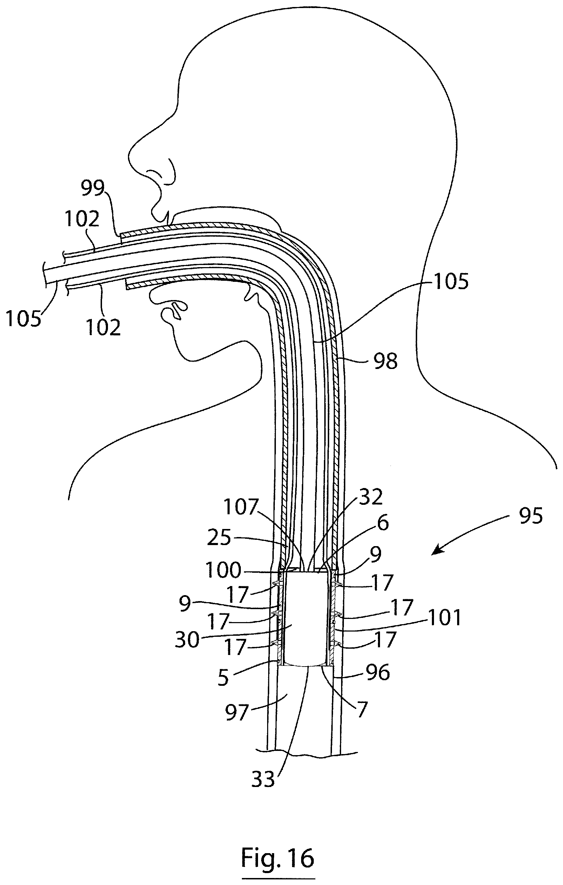

With the carrier element 5 fully exposed in the oesophagus 97, the operating member 30 is urged by an endoscope 105 or other suitable urging device through the carrier element accommodating bore 25 of the tubular shield 98 to the carrier element 5, see Fig. 15. The proximal end 32 of the operating member 30 is secured to a distal end 107 of the endoscope. With the distal end 33 of the operating member 30 adjacent the proximal end 6 of the main bore 14 of the carrier element 5, the operating member 30 is urged by the endoscope 105 into the main bore 14 of the carrier element 5 for in turn urging the shells 9 of the carrier element 5 transversely outwardly of the main axis 15 of the carrier element 5, from the rest state to the engagement state, for in turn urging the microneedles 17 into the wall 96 of the oesophagus 97 on opposite sides thereof, see Fig. 16. The operating member 30 is urged into the main bore 14 of the carrier element 5 until the communicating ports 45 in the operating member 30 are aligned with the inlet ports 21 of the shells 9 for communicating the main chambers 40 of the operating member 30 with the corresponding inlet ports 21 in the shields 9 of the carrier element 5. The main chambers 40 and the communicating ports 45 of the operating member 30 are not illustrated in the drawings of this embodiment of the invention, but are similar to the main chambers 40 and the communicating ports 45 of the operating member 30 of the microneedle device 1 described with reference to Figs. 1 to 8.

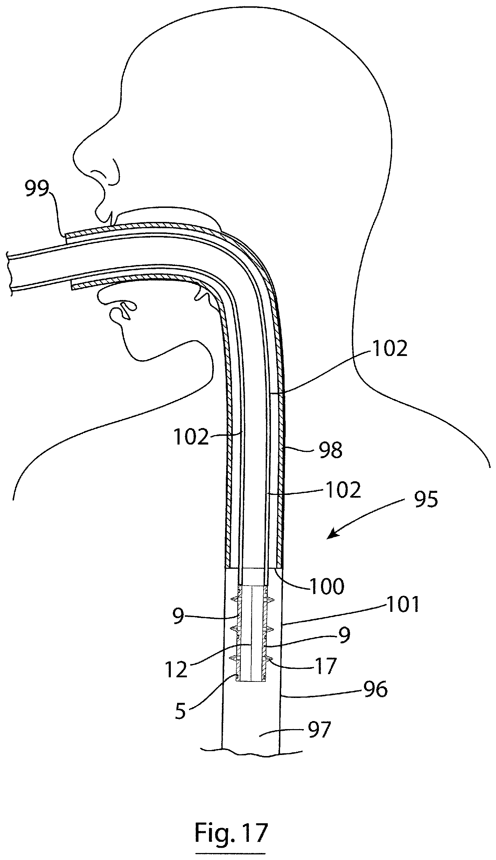

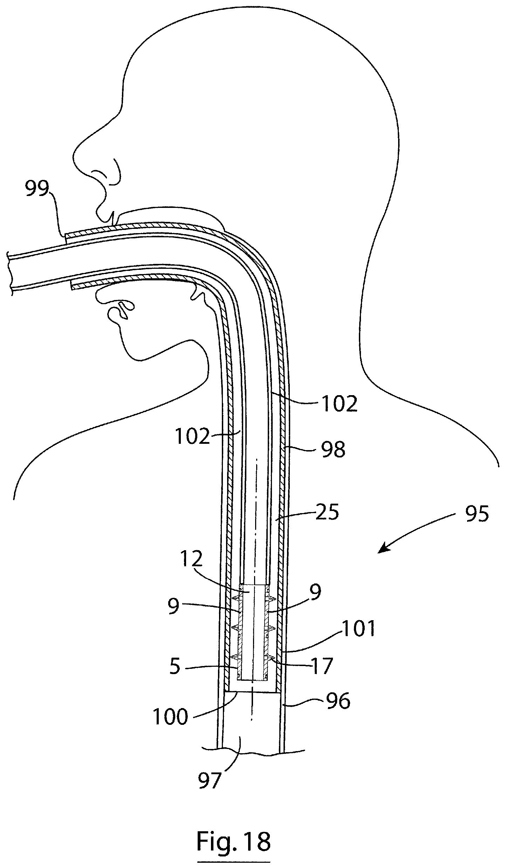

With the microneedles 17 engaged in the wall 96 of the oesophagus 97, and the communicating ports 45 aligned with the inlet ports 21 of the corresponding microneedles 17, the delivery needle 52 is introduced through the endoscope 105 and in turn is inserted into the operating member 30 for sequentially delivering the therapeutic liquid into the main chambers 40a to 40c as already described with reference to the microneedle device 1 described with reference to Figs. 1 to 8. On completion of the treatment of the wall 96 of the oesophagus 97 with the therapeutic liquid, with the carrier element 5 held in place by the carrier members 102, the operating member 30 is withdrawn by the endoscope 105 through the carrier element accommodating bore 25 of the tubular shield 98, see Fig. 17. With the carrier element 5 held in place by the carrier members 102, the tubular shield 98 is urged downwardly in the oesophagus over the carrier element 5 until the carrier element 5 and the microneedles 17 are located entirely within the carrier element accommodating bore 25 of the tubular shield 98 adjacent the distal end 100 thereof, see Fig. 18. With the carrier element 5 and the microneedles 17 safely located within the tubular shield 98, the tubular shield 98 with the carrier element 5 located therein is then withdrawn from the oesophagus.

It will be appreciated that suitable alignment means for circumferentially aligning the operating member 30 with the carrier element 5 in the main bore 14 and also for longitudinally aligning the operating member 30 in the main bore 14 of the carrier element 5 will be provided in the microneedle device 95. Such alignment means for circumferentially aligning the operating member 30 in the main bore 14 of the carrier element 5 so that the communicating ports 45 are circumferentially aligned with the inlet ports 21 may, for example, comprise a keying system. Such a keying system may comprise a keying ridge slideably engageable in a longitudinally extending keying groove, one of which may be provided on one or both of the shells 9 of the carrier element 5 and the other one or ones of which would be provided on the operating member 30. A longitudinal alignment means for longitudinally aligning the operating member 30 with the carrier element 5 may be provided, for example, by a pair of projections extending from the operating member 30 on respective diametrically opposite sides thereof or from the shells of the carrier element, which would be engageable with corresponding recesses formed in the inner surface 49 of the shells 9 of the carrier element 5 or the operating member as appropriate. The projections, could be circular projections or transversely extending ridges, which would engage correspondingly shaped recesses in the shells 9 of the carrier element 5. Needless to say, the projections may be provided on the shells 9 for engaging corresponding recesses on the operating member 30. It is also envisaged that visual alignment means may be provided for both longitudinal and circumferential alignment which could be viewed by a camera on the end of the endoscope for alignment of the operating member 30 with the carrier element 5. Such visual alignment means may be similar to the longitudinal and circumferential alignment means of the microneedle device 1 of Figs. 1 to 8.

The lengths of the microneedles 17 will be determined so that the therapeutic liquid is injected into the wall 96 of the oesophagus 97 at a suitable depth into the wall, and the lengths of the microneedles 17 will be such as to avoid any danger of the microneedles extending completely through the wall 96 of the oesophagus 97.

Otherwise the microneedle device 95 and its use is similar to that of the microneedle device 1 described with reference to Figs. 1 to 8.

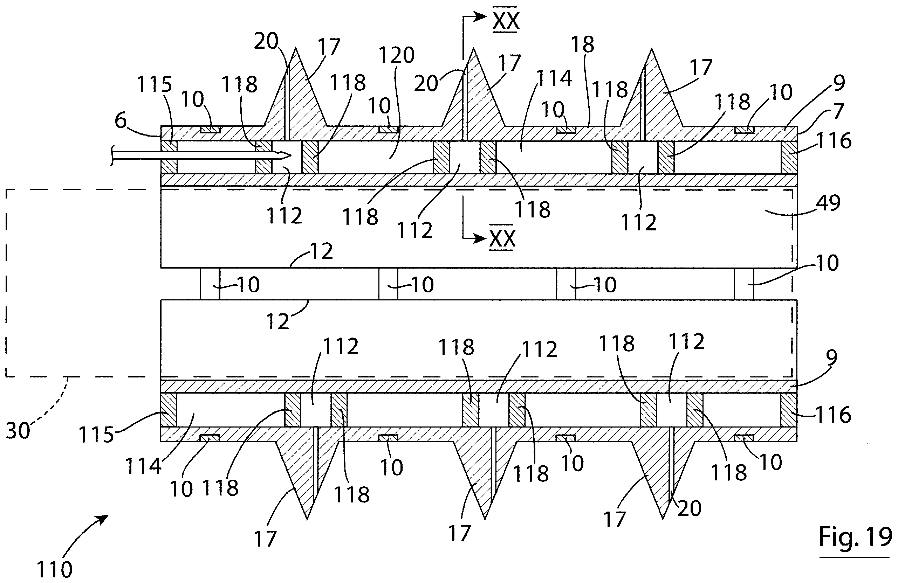

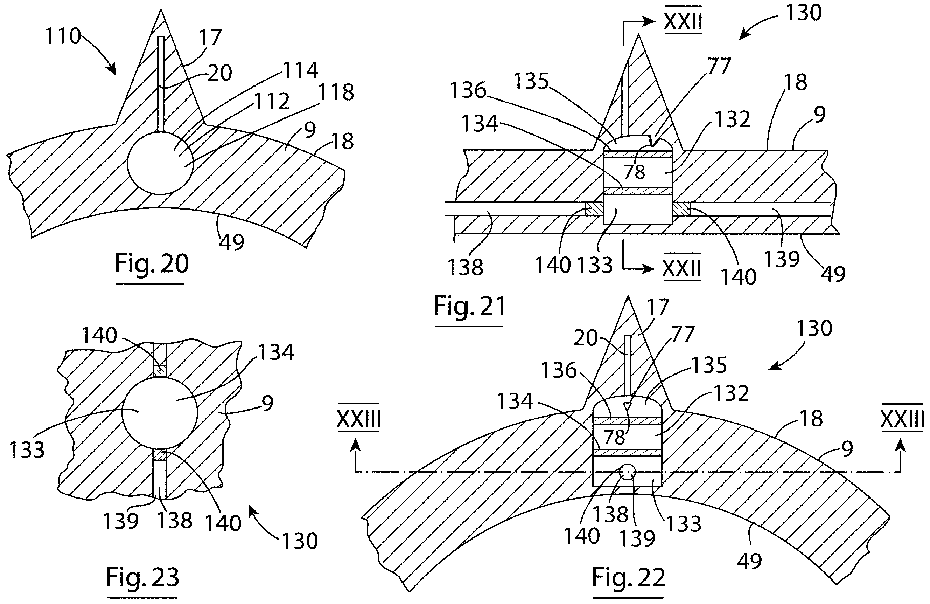

Referring now to Figs. 19 and 20, there is illustrated a portion of a microneedle device according to the invention indicated generally by the reference numeral 110. The microneedle device 110 according to this embodiment of the invention is somewhat similar to the microneedle device 1, described with reference to Figs. 1 to 8, and similar components are identified by the same reference numerals. The main difference between the microneedle device 110 and the microneedle device 1 is that firstly, the operating member 30 has no function other than to urge the shells 9 of the carrier element 5 apart from the rest state to the engagement state when the carrier element 5 has been located in the fistula or other lumen, vessel, cavity, organ or muscular organ in the desired location.

In this embodiment of the invention the main chambers for accommodating the therapeutic fluid to be delivered to the subject are located in the shells 9 of the carrier element 5, and are identified by the reference numeral 112. The main chambers 112 corresponding to each group of axially aligned microneedles 17 are formed in the corresponding shell 9 by a corresponding elongated needle accommodating bore 114 of circular transverse cross-section extending through the corresponding shell 9 from the proximal end 6 thereof to the distal end 7. The bore 114 in each shell 9 is sealably closed at the proximal end thereof by a proximal end plug 115, and at the distal end by a distal end plug 116. The proximal and distal end plugs 115 and 116 are substantially similar to the proximal end plug 37 and the distal end plug 36 of the microneedle device 1 of Figs. 1 to 8. The main chambers 112 of each shell 9 are formed in and are sealably isolated from the corresponding bore 114 by second sealing means comprising spaced apart pairs of spaced apart intermediate plugs 118 located in the bore 114. The intermediate plugs 118 are located in the bore 114 of the corresponding shell 9 to form the main chambers 112 in the bore 114 so that the main chambers 112 communicate with the needle accommodating bore 20 of the corresponding microneedles 17. The proximal and intermediate plugs 115 and 118 are of a substantially similar puncturable, self-resealing sealing material as the proximal and intermediate plugs 37 and 39 of the microneedle device 1. The bore 114 in each shell 9 of the carrier element 5 forms a needle accommodating duct for accommodating a delivery needle 52 of a needle and syringe assembly 50, similar to that described with reference to the microneedle device 1, sequentially to the main chambers 112, in a similar manner as the needle accommodating duct 38 formed by the bore 35 extending through the operating member 30 of the microneedle device 1 accommodates the delivery needle 52 sequentially into the main chambers 40 of the operating member 30 of the microneedle device 1.

The shells 9 of the carrier element 5 are resiliently urged together by four bands 10 of resilient material similar to the bands 10 of the microneedle device 1 and in a similar manner as the bands 10 of the microneedle device 1 urge the shells 9 of the carrier element 5 of the microneedle device 1 together.

In this embodiment of the invention since the main chambers 112 and the needle accommodating ducts 120 are located in the shells 9 of the carrier element 5 as discussed above, the operating member 30 has only one function, namely, to urge the shells 9 of the carrier element 5 apart from the rest state to the engagement state, for in turn urging the microneedles 17 from the rest state to the engagement state into the tissue of a subject. Accordingly, in this embodiment of the invention the operating member 30 may be a solid member, or may be a tubular member similar to that described with reference to the microneedle device 1, with the exception that the main chambers 40 may be omitted. Alternatively, the operating means in this embodiment of the invention instead of being provided by a solid member, may be provided by an inflatable member, for example, a balloon of a balloon catheter for urging the shells 9 apart as the balloon is inflated.

Finger grip elements (not shown) but similar to the finger grip elements 22 of the microneedle device 1 described with reference to Figs. 1 to 8 are releasably coupled to the shells 9 of the carrier element 5 of the microneedle device 110 by quick release couplers for positioning the carrier element 5 in the fistula, lumen, vessel or cavity at the desired location as described with reference to the microneedle device 1 of Figs. 1 to 8.

Use of the microneedle device 110 is similar to that of the microneedle device 1, with the exception that when the microneedle device 110 has been located in the fistula, lumen, vessel or cavity and the tubular shield has been removed, the operating member is urged into the main bore 14 of the carrier element 5 for urging the shells 9 apart, for in turn urging the microneedles 9 into the tissue adjacent the fistula, lumen, vessel or cavity as already described with reference to the microneedle device 1. The delivery needle 52 is then sequentially urged into the main chambers 112 through the corresponding needle accommodating duct 120 of the respective shells 9 of the carrier element 5 for in turn delivering the therapeutic fluid into the main chambers 112 and in turn through the fluid accommodating bores 20 of the microneedles 17 to the tissue adjacent the fistula, lumen, vessel or cavity of the subject as already described with reference to the microneedle device 1.

Referring now to Figs. 21 to 23, there is illustrated a portion indicated generally by the reference numeral 130 of a microneedle device according to another embodiment of the invention. The microneedle device according to this embodiment of the invention is somewhat similar to the microneedle device 1, and similar components are identified by the same reference numerals. However, in this embodiment of the invention like in the microneedle device 110, main chambers 132 are located in the shells 9 of the carrier element 5, and the main chambers 132 are configured to store the therapeutic fluid. In this embodiment of the invention each main chamber 132 is sized to store a single dose of the therapeutic fluid. Additionally, in this embodiment of the invention a secondary chamber 133 corresponding to each main chamber 132 is also located in the shells 9 of the carrier element 5. Each main chamber 132 and its corresponding secondary chamber 133 are of cylindrical shape and are isolated from each other by a main membrane 134 similar to the main membrane described with reference to the microneedle device of Figs. 10 and 11. Additionally, each microneedle 17 is provided with an intermediate chamber 135 between the main chamber 132 and the needle accommodating bore 20 of the microneedle 17. The main chamber 132 of each microneedle 17 is isolated from the corresponding intermediate chamber 135 by a secondary membrane 136. The secondary membrane 136 is substantially similar to the secondary membranes 74 described in the microneedle devices 70 and 80 described with reference to Figs. 10 and 11. A piercing member 77 similar to the piercing member 77 of the microneedle devices 70 and 80 described with reference to Figs. 10 and 11 extend from each microneedle 17 into the corresponding intermediate chamber 135 for rupturing the secondary membrane 136 when the secondary membrane 136 is urged against the piercing point 78 of the piercing member 77.

A needle accommodating duct 138 formed by an elongated bore 139 extends through each shell 9 of the carrier element 5 for accommodating a delivery needle 52 similar to the delivery needle 52 of the needle and syringe assembly 50 described with reference to the microneedle device 1, sequentially into the secondary chambers 133 for delivering pressurised air or other gas into the secondary chambers 133 for in turn urging the main membrane 134 into the corresponding main chamber 132, for in turn rupturing the corresponding secondary membrane 136 and urging the therapeutic fluid in the main chamber 132 through the needle accommodating bore 20 of the corresponding microneedle 17.

Each secondary chamber 133 is isolated from the needle accommodating duct 138 by a corresponding pair of spaced apart intermediate sealing plugs 140. The intermediate sealing plugs 140 are similar to the intermediate sealing plugs 39 described with reference to the microneedle device 1, and are of a puncturable resilient self-resealing material. The intermediate sealing plugs 140 accommodate the delivery needle 52 therethrough and reseal themselves on withdrawal of the delivery needle 52.

In this embodiment of the invention since both the main chambers 132 and the secondary chambers 133 are located in the shells 9 of the carrier element 5, the only function of the operating member 30 (not shown) is to urge the shells 9 of the carrier element 5 apart from the rest state to the engagement state in order to urge the microneedles 17 into the tissue adjacent the fistula, lumen, vessel, cavity, organ or muscular organ of the subject. Accordingly, like the microneedle device 110, the operating member may be a solid member, or an inflatable member.

Use of the microneedle device according to this embodiment of the invention is substantially similar to that of the microneedle device 110, with the exception that instead of delivering the therapeutic fluid into the main chambers by the delivery needle 52, as in the case in the microneedle device 110, in the case of the microneedle device 130, when the carrier element is located in the fistula, lumen, vessel, cavity, organ or muscular organ with the microneedles having been urged into the adjacent tissue by the operating member, the delivery needle 52 is sequentially urged into the secondary chambers 133, and air or other gas is delivered under pressure by the syringe 51 of the needle and syringe assembly 50 into the secondary chambers 133 for in turn discharging the therapeutic fluid from the main chambers 132 through the needle accommodating bores 20 of the corresponding microneedles 17 to the tissue of the subject.

Otherwise, the microneedle device 130 and its use is similar to the microneedle device 1 and its use.

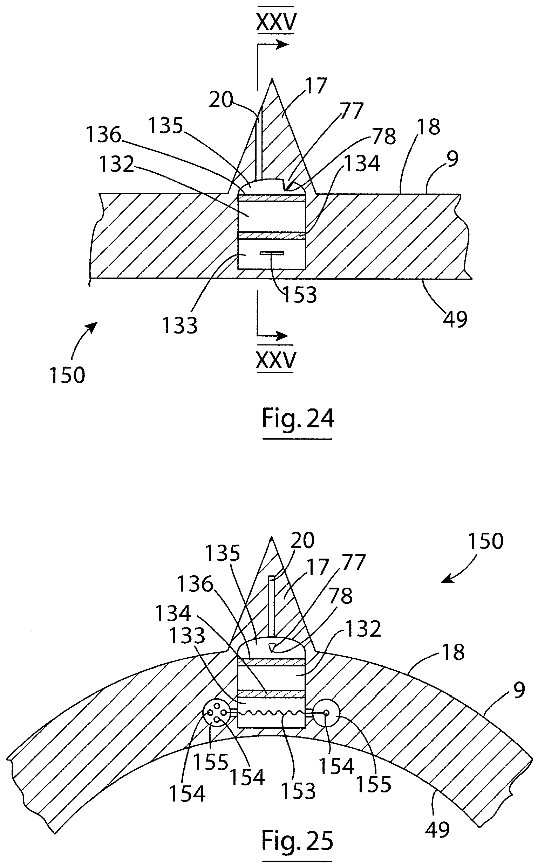

Referring now to Figs. 24 and 25, there is illustrated a portion of a microneedle device according to another embodiment of the invention indicated generally by the reference numeral 150. The microneedle device 150 according to this embodiment of the invention is somewhat similar to the microneedle device 1, and is also substantially similar to the microneedle device 130, and similar components are identified by the same reference numerals. The only difference between the microneedle device 150 and the microneedle device 130 lies in the secondary chambers 133. In this embodiment of the invention the main chambers 132 and the secondary chambers 133 are isolated from each other by main membranes 134, which are similar to the main membrane 72 of the microneedle device 70. The main chambers 132 are isolated from the corresponding intermediate chambers 135 by secondary membranes 136 which are similar to the secondary membrane 74 of the microneedle device 70. However, in this embodiment of the invention instead of the secondary chambers 133 being provided to accommodate pressurised air or other gas for in turn urging the main membranes 134 into the main chambers 132, the secondary chambers 133 contain an expandable medium, in this case microbeads of the type which expand to a multiple of their size, and are similar to the microbeads located in the secondary chamber 83 of the microneedle device 80. Heating elements 153, which are similar to the heating elements 85 of the microneedle device 80 are located in the secondary chamber 133 for heating the microbeads therein. The heating elements 153 in the secondary chambers 133 are individually and independently electrically powered by electrically conductive wires 154 extending through elongated wire accommodating bores 155 which extend longitudinally through the shells 9 from the proximal end 6 thereof to the respective heater elements opposite sides of the secondary chambers 133. The wires 154 at the proximal ends 6 of the shells 9 are connected to a suitably switched electrical power supply.

Since both the main chambers 132 and the secondary chamber 133 are located in the shells 9 of the carrier element 5, and the electrically conductive wires 154 for powering the heating elements 153 extend through the wire accommodating bores 155 located in the shells 9, the only function of the operating member (not shown) is to urge the shells 9 apart from the rest state to the engagement state as already described with reference to the operating member 30 of the microneedle device 1. Accordingly, the operating member (not shown) may be provided by a solid member or an inflatable member, as for example, a balloon of a balloon catheter described with reference to the microneedle device 110.

Use of the microneedle device 150 is similar to that of the microneedle device 130, with the exception that instead of pressurising the secondary chambers 133 with air or other gas through the delivery needle 52, once the microneedle device 150 has been located in the fistula, lumen, vessel, cavity, organ or muscular organ, with the microneedles 17 engaged in the tissue, the electrical supply is applied to the heating elements 153 in the secondary chambers 133 either sequentially or simultaneously, or in pairs of the secondary chambers 133 or in any other desired sequence.

It is envisaged that in the embodiment of the invention described with reference to Figs. 10, 11 and 19 to 25, the shells 9 of the microneedle devices 70, 80, 110, 130 and 150 or a part thereof comprising the microneedles, the main chambers, the secondary chambers and the intermediate chambers, may be constructed in layers as for example disclosed in PCT Specification No. WO 2010/052692 of O'Dea et al.

It is envisaged that in some embodiments of the invention, the main chambers in the operating member 30, may be configured to store the therapeutic fluid or other drug or fluid. In which case, it is envisaged that the main chambers would be isolated from the communicating ports by flexible elastically expandable membranes which would retain the therapeutic fluid until the membranes are ruptured. It is envisaged that a rupturing system would be provided in the operating member similar to that provided in the microneedle devices 70, 80, 110, 130 and 150 whereby a piercing member would be provided for piercing the corresponding membrane on the therapeutic fluid in the main chamber being pressurised. It is also envisaged that in such an embodiment of the invention, the operating member 30 would be provided with an urging means corresponding to each main chamber for urging the fluid in the main chamber out of the main chamber and through the corresponding microneedle. Such an urging means, could typically be provided by microbeads similar to those described with reference to the microneedle device 80, which would be located in a secondary chamber adjacent the corresponding main chamber, and isolated from the corresponding main chamber by a main membrane, similar to the main membrane 72 of the microneedle device 80. An electrically powered heating element would be provided in each secondary chamber for heating the microbeads therein. To expand the microbeads, and in turn urge the main membranes into the corresponding main chambers, an electrical power supply would be provided to each heating element. Expanding of the microbeads by heating thereof in each secondary chamber would result in rupturing of the corresponding membranes isolating the corresponding main chambers from the communicating ports thereof and in turn urging the therapeutic fluid stored in the corresponding main chamber through the fluid accommodating bores 20 in the corresponding microneedles 17, and in turn into the tissue of the subject adjacent the fistula, or the wall or tissue of the lumen, vessel, cavity, organ or muscular organ of the subject to be treated.

The dimensions of the carrier element 5, the microneedles 17, the operating member 30, the tubular shield 24 and the tubular shield 98 of the microneedle devices will be largely dependent on the uses to which the microneedle devices according to the invention are to be put, as will be understood by those skilled in the art.

While the carrier element 5 of each microneedle device has been described as comprising a pair of shells which are resiliently urged together to form a cylindrical carrier element 5, it is envisaged that any other suitable construction of carrier element 5 may be provided. Indeed, it is envisaged that in certain embodiments of the invention the shells 9 of the carrier elements 5 may be sealably connected along their respective edges 12 by a resilient expandable membrane. Indeed, it is envisaged that in some embodiments of the invention the carrier element 5 may comprise an elastically expandable material which would expand on the operating member being urged through the main bore thereof in order to urge the microneedles into the tissue of a subject.

While the carrier element of each of the microneedle devices which have been described, have been described as comprising a plurality of microneedles, it is envisaged that in some embodiments of the invention only a single microneedle may be provided. It is also envisaged that where the carrier element is provided by a pair of shells or a resiliently expandable tubular element, only one, or two microneedles may be provided. For example, only a single microneedle may be provided on one of the shells only, or two or more microneedles may be provided on one of the shells only, and no microneedles may be provided on the other shell. It is also envisaged that the microneedles may be located on one or on respective opposite sides of the carrier element. It is also envisaged that where more than one microneedle is provided on the carrier element, each main chamber of the operating member 30 may be alignable with one microneedle only.

It will of course be appreciated that in order to minimise wastage of the therapeutic fluid, the main chambers of the microneedle devices, in which the main chambers are configured for accommodating the flow of the therapeutic fluid passing therethrough to the microneedles, will be of minimal size just sufficient to accommodate the distal end 56 and the side port 54 of the delivery needle 52. It will also be appreciated that the cross-sectional area of the inlet ports 21 will be of minimal size, and will be of size just sufficient for alignment with the corresponding communicating ports 45 of the operating member 30.

While the shielding means of each microneedle device has been described as comprising a tubular shield having a carrier element accommodating bore extending therethrough, it is envisaged that instead of providing a separate tubular shield as the tubular shields 24 or 98, the shielding means may be provided by a shielding element or two shielding elements which would be provided with needle accommodating bores for accommodating the microneedles therethrough, and such shielding elements would be moveable relative to the carrier element 5 from a shielding position spaced apart from the carrier element 5 shielding the microneedles 17 to a non-shielding position adjacent the carrier element 5 with the microneedles extending through the microneedle accommodating bores and outwardly from the shielding element.

It is also envisaged that when the shielding means is provided as a tubular shield, that the tubular shield may be provided with microneedle accommodating openings therethrough for accommodating the microneedles through the openings as the shells of the carrier element are urged by the operating means from the rest state to the engagement state. In other words, the microneedle accommodating openings would extend radially through the tubular shield. The outer diameter of the tubular shield would be such that when the microneedles are in the rest state, the microneedles would be recessed into the microneedle accommodating openings, and would not project beyond the external surface of the tubular shield. When the microneedles are operated into the engagement state, the microneedles would extend outwardly through the microneedle accommodating openings beyond the external surface of the tubular shield to engage and extend into the tissue into which the therapeutic fluid is to be injected. In this case, the tubular shield would not have to be withdrawn from the lumen, vessel, cavity or fistula prior to engagement of the operating member in the main bore of the carrier element. The tubular shield with the carrier element in the carrier element accommodating bore, and with the microneedles in the rest state, would be inserted into the lumen, vessel, cavity or fistula, and when the tubular shield and the carrier element are correctly located in the lumen, vessel, cavity or fistula, the operating member would then be urged into the main bore of the carrier element to urge the microneedles from the rest state through the microneedle accommodating openings in the tubular shield to the engagement state for engaging and extending into the tissue to be injected with the therapeutic fluid. On completion of the treatment of the site with the therapeutic fluid, the operating member would be withdrawn from the carrier element, thereby returning the microneedles from the engagement state to the rest state within the microneedle accommodating openings of the tubular shield. The tubular shield, with the carrier element located in the carrier element accommodating bore of the tubular shield, and the microneedles in the rest state, would then be withdrawn from the lumen, vessel, cavity or fistula.

Needless to say it will be appreciated that while some of the microneedle devices have been described for use in the treatment of a fistula with a therapeutic fluid which may comprise stem cells, those microneedle devices may be used for treating any fistula, be it an anal fistula or any other fistula with any other suitable therapeutic material besides stem cells, for example, the treatment could comprise a drug therapy or indeed any other fluid treatment.

It will also be appreciated that while the microneedle device according to the invention described with reference to Figs. 13 to 18 has been described for use in a method for injecting the wall of an oesophagus with a therapeutic liquid, that microneedle device and others of the microneedle devices may be used for injecting any drug, fluid, stem cells or any other liquid or fluid into the wall or tissue adjacent any other lumen, vessel, cavity, organ or muscular organ in a human or animal subject be it an artery, a vein, the intestine, the urethra, the bowel, the rectum or any other lumen, vessel, cavity, organ or muscular organ.

While the microneedle device described with reference to Figs. 13 to 18 has been described as comprising a carrier element and an operating member similar to that described with reference to Figs. 1 to 8, it will be appreciated that the microneedle device may comprise any of the carrier elements and the operating members described herein with reference to Figs. 1 to 12 and Figs. 19 to 25. It is also envisaged that the tubular shield of the microneedle device of Figs. 13 to 18, may terminate in a distal portion comprising microneedle accommodating openings similar to those described above for accommodating the microneedles therethrough.

It will also be appreciated that while each main chamber in the operating member has been described as comprising two communicating ports therefrom, in certain embodiments of the invention only a single communicating port may be provided from each main chamber, and this would be so in cases where only a single microneedle was provided corresponding to each main chamber. Needless to say, any number of main chambers may be provided in the operating member 30 from one upwards, and in general, the number of main chambers both in the operating member and in the carrier element will be dependent on the number of microneedles or pairs of microneedles. In some embodiments of the invention a single main chamber in the operating member may be sufficient, and the operating member 30 would be urged along the main bore 14 of the carrier element 5 for sequentially aligning the main chamber with respective ones of the microneedles or respective pairs of the microneedles as the case may be.

It will be readily apparent to those skilled in the art that while the operating member has been described as comprising a tubular member, while this is a convenient method for producing the operating member, it is not essential that the operating member be tubular, indeed, the operating member may be a solid member, with suitable bores formed therein. Indeed, it is also envisaged that the operating member may comprise an expandable element, such as a mechanically reversibly expandable element or an inflatable element, such as a balloon, and in which case the expandable element would be inserted in the main bore of the carrier element in the unexpanded state and would then be expanded to urge the microneedle or microneedles, as the case may be, into the wall or tissue of the lumen, vessel, cavity or fistula.

It will also of course be appreciated that while the carrier element has been described as being of cylindrical shape, and the operating member has also been described as being of circular transverse cross-section, and effectively, being of cylindrical shape, the carrier element and the operating member may be of any other suitable shape, for example, the carrier element and the operating member may be of square, rectangular, triangular, hexagonal or any desirable polygonal transverse cross-section. It will also be appreciated that the operating member and the carrier element may be of respective different transverse cross-sections.

While the delivery needle for delivering the therapeutic fluid into the main chambers or for delivering pressurised air or other gas into the secondary chambers has been described as delivering the therapeutic fluid or air or other gas into the main chambers or the secondary chambers in a particular sequence, the therapeutic fluid or air or other gas may be delivered into the main chambers or the secondary chambers in any desired sequence. It is also envisaged that an in-built delivery means may be provided within the operating member or the carrier element as the case may be for delivering the therapeutic fluid or air or other gas to the main chambers or the secondary chambers, for example, individual lumens may be provided in the operating member or the carrier element which would communicate with the respective main chambers or secondary chambers.

It will also be appreciated that while the operating members have been described as comprising three main chambers, the operating members may be provided with any number of main chambers, and the number of main chambers, in general, will be dependent on the number of microneedles or pairs of microneedles on the carrier element.

It is also envisaged that in the microneedle device 95 described with reference to Figs. 13 to 18, the carrier element 5 of the microneedle device 95 may be provided with shells 9 similar to the shells 9 of the microneedle devices 70, 80, 110, 130 and 150 described with reference to Fig. 10, 11, and 19 to 25.

It is also envisaged that as well as the shells 9 being provided with secondary chambers 83 corresponding to the main chambers, and with heater elements 85 located in the secondary chambers 83, wires coupling the heating elements 85 to a suitable switched electrical power supply could be located within the shells 9, and would extend from the proximal ends of the shells 9 to the switched electrical power supply, as described with reference to the microneedle device 150 of Figs. 24 and 25. In which case, the operating member would be provided as solid or an expandable operating member, and its function would be solely for the purpose of urging the shells 9 of the carrier element 5 transversely outwardly of the main axis 15 of the carrier element 5 from the rest state thereof to the engagement state thereof for in turn urging the microneedles 17 from the rest state to the engagement state into engagement with the wall 96 of the oesophagus 97, or into engagement with a wall or tissue of any other lumen, vessel, cavity, organ or muscular organ.

While the tubular shield of the microneedle device 95 described with reference to Figs. 13 to 18 has been described as being of a transparent material, while this is desirable, it is not essential.

In cases where the microneedle device 95 is being used for treating the wall of the intestine, the colon, the bowel or the rectum, it is envisaged that a guide wire would initially be entered endoscopically to the location to be treated, and the tubular shield would then be slid over the guide wire, the guide wire would then be withdrawn and the carrier element with the operating member removed would be urged through the tubular shield to the distal end thereof. Thereafter, operation of the microneedle device 95 would be similar to that already described for use in treating the wall of the oesophagus with a therapeutic fluid.

It will also be appreciated that any other suitable gripping means for gripping and holding the shells of the carrier element may be provided besides the finger grip elements already described. Indeed, it is envisaged that in some embodiments of the invention where finger grip elements are provided, they may be of a suitable size and shape which would avoid the need to disengage them from the shells of the carrier element for removal of the tubular shield.

It is envisaged that any other materials may be employed in the microneedle devices described, besides the materials described herein.

While the fluid accommodating bores extending through the respective microneedles have been described as extending substantially centrally through and coaxially with the microneedles, it is envisaged that in some embodiments of the invention the fluid accommodating bores may extend through the microneedles offset from the microneedle axis, but typically, although not necessarily would extend parallel to the microneedle axis. Additionally, while the microneedles have been described as being of length of 1mm to 2mm, it will be appreciated that the microneedles may be of any suitable length, and the length of the microneedles will be chosen depending on the depth to which the therapeutic fluid or liquid, the microbeads or other bulking agents or substances are to be injected in the tissue, wall or musculature of the lumen, vessel, cavity, organ, muscular organ or fistula. However, in general, it is envisaged that the length of the microneedles will lie in the range of 0.5mm to 10mm, and more particularly in the range of 1mm to 5mm, although the length of the microneedles may be longer or shorter than these ranges.

The microneedle devices according to the invention may be used for delivering any fluid or liquid, whether the fluid or liquid is a therapeutic fluid or liquid or otherwise. It has been found that as well as being suitable for delivering fluids and liquids containing stem cells, drugs and other such substances, the microneedle devices according to the invention are also particularly suitable for delivering microbeads and other bulking agents and substances submucosally to muscular organs, for example, sphincters, such as the lower oesophageal sphincter for improving the sealing function of the sphincter in the treatment of gastroesophageal reflux disease.

## Claims

1. A microneedle device comprising a carrier element (5) defining a longitudinally extending main axis (15), at least one microneedle (17) defining a needle axis (19), the at least one microneedle (17) being carried on the carrier element (5) with the needle axis (19) extending substantially transversely relative to the main axis (15) defined by the carrier element (5), and an operating means (30) co-operable with one of the carrier element (5) and the at least one microneedle (17) for urging the microneedle (17) transversely outwardly relative to the main axis (15) from a rest state to an engagement state for engaging tissue, **characterised in that** one of the carrier element (5) and the operating means (30) is configured to accommodate a communicating means (35, 40, 112, 120) for communicating each microneedle (17) with a source (51) of the fluid to be delivered to a subject through the microneedle device, and the communicating means (35, 40, 112, 120) comprises at least one main chamber (40, 112) located in the one of the carrier element (5) and the operating means (30), each main chamber (40, 112) being configured to accommodate the fluid to be delivered to a subject and to communicate with the corresponding one or more of the microneedles.

2. A microneedle device as claimed in Claim 1 **characterised in that** the carrier element (5) defines a main bore (14) extending longitudinally into the carrier element (5) from a proximal end (6) thereof, the operating means (30) being engageable in the main bore (14) defined by the carrier element (5) and being configured for increasing the cross-section of the main bore (14) defined by the carrier element (5).

3. A microneedle device as claimed in Claim 1 or 2 **characterised in that** the operating means (30) comprises an operating member, the operating member (30) being of non-deformable transverse cross-section.

4. A microneedle device as claimed in any preceding claim **characterised in that** the carrier element (5) comprises a pair of shells (9).

5. A microneedle device as claimed in Claim 4 **characterised in that** the shells (9) are resiliently urged towards each other into a rest state thereof for retaining the at least one microneedle (17) in the rest state thereof.

6. A microneedle device as claimed in any preceding claim **characterised in that** each main chamber (40, 112) is configured to accommodate fluid passing therethrough from the fluid source (57) to the corresponding one or more of the microneedles (17).

7. A microneedle device as claimed in any preceding claim **characterised in that** the main chamber (40), 112) is configured to communicate with a fluid delivery means (52) configured to deliver a fluid from a remote source (51) to the main chamber (40, 112).

8. A microneedle device as claimed in Claim 7 **characterised in that** the fluid delivery means (52) comprises an elongated fluid delivery needle having a fluid accommodating bore (55) extending therethrough to a distal end (56) thereof.

9. A microneedle device as claimed in Claim 8 **characterised in that** the one of the carrier element (5) and the operating means (30) comprises a fluid delivery needle accommodating duct (35, 120) for accommodating the fluid delivery needle (52) therethrough to the main chamber (40, 112).

10. A microneedle device as claimed in Claim 9 **characterised in that** each main chamber (40, 112) is isolated from the fluid delivery needle accommodating duct (35, 120) by a first sealing element (29, 118), and each first sealing element (39, 118) is configured to sealably accommodate the fluid delivery needle (52) therethrough to the corresponding one of the main chambers (40, 112).

11. A microneedle device as claimed in Claim 10 **characterised in that** each first sealing element (39, 118) comprises a self-resealing sealing element configured to reseal itself on withdrawal of the fluid delivery needle (52).

12. A microneedle device as claimed in any preceding claim **characterised in that** each main chamber (40, 112) is located in the operating means (30), and the operating means (30) is alignable with the carrier element (5) for aligning the at least one main chamber (40, 112) with a corresponding one or more of the microneedles (17) for communicating the main chamber (40, 112) with a fluid accommodating bore (20) of each microneedle (17) corresponding to the main chamber (40, 112).

13. A microneedle device as claimed in any preceding claim further comprising a shielding means (24, 98) for protecting the microneedles (17).

14. A microneedle device as claimed in Claim 13 **characterised in that** the shielding means (24, 98) comprises a tubular shield (24, 98) having a carrier element accommodating bore (25) extending therethrough for accommodating the carrier element (5) and the microneedles (17) therein when the microneedles are in the rest state.

15. A microneedle device as claimed in any preceding claim for use in a method for the treatment of a site in a human or animal body with an injectable fluid.

## Patentansprüche

1. Mikronadelvorrichtung, umfassend ein Trägerelement (5), das eine sich longitudinal erstreckende Hauptachse (15) definiert, mindestens eine Mikronadel (17), die eine Nadelachse (19) definiert, wobei die mindestens eine Mikronadel (17) auf dem Trägerelement (5) getragen wird, wobei sich die Nadelachse (19) im Wesentlichen transversal in Bezug auf die durch das Trägerelement (5) definierte Hauptachse (15) erstreckt, und ein Betätigungsmittel (30), das mit einem von dem Trägerelement (5) und der mindestens einen Mikronadel (17) zusammenwirken kann, um die Mikronadel (17) in Bezug auf die Hauptachse (15) von einem Ruhezustand in einen Eingriffszustand zum Eingriff mit Gewebe transversal nach außen zu drücken, **dadurch gekennzeichnet, dass** eines von dem Trägerelement (5) oder dem Betätigungsmittel (30) so konfiguriert ist, dass es ein Verbindungsmittel (35, 40, 112, 120) aufnimmt, um jede Mikronadel (17) mit einer Quelle (51) der Flüssigkeit zu verbinden, die durch die Mikronadelvorrichtung an eine Person abgegeben werden soll, und das Verbindungsmittel (35, 40, 112, 120) mindestens eine Hauptkammer (40, 112) umfasst, die sich in dem einen von Trägerelement (5) und Betätigungsmittel (30) befindet, wobei jede Hauptkammer (40, 112) derart konfiguriert ist, dass sie die einer Person zu verabreichende Flüssigkeit aufnimmt und mit den entsprechenden ein oder mehreren Mikronadeln verbunden ist.

2. Mikronadelvorrichtung wie in Anspruch 1 beansprucht, **dadurch gekennzeichnet, dass** das Trägerelement (5) eine Hauptbohrung (14) definiert, die sich in longitudinaler Richtung in das Trägerelement (5) von einem proximalen Ende (6) hiervon erstreckt, wobei das Betätigungsmittel (30) in die durch das Trägerelement (5) definierte Hauptbohrung (14) eingreifbar ist und konfiguriert ist zum Vergrößern des Querschnitts der Hauptbohrung (14), die durch das Trägerelement (5) definiert ist.

3. Mikronadelvorrichtung wie in Anspruch 1 oder 2 beansprucht, **dadurch gekennzeichnet, dass** das Betätigungsmittel (30) ein Betätigungselement umfasst, wobei das Betätigungselement (30) einen nicht verformbaren transversalen Querschnitt aufweist.

4. Mikronadelvorrichtung wie in einem der vorhergehenden Ansprüche beansprucht, **dadurch gekennzeichnet, dass** das Trägerelement (5) ein Paar Hüllen (9) umfasst.

5. Mikronadelvorrichtung wie in Anspruch 4 beansprucht, **dadurch gekennzeichnet, dass** die Hüllen (9) durch Federkraft zueinander in einen Ruhezustand gedrückt werden, um die mindestens eine Mikronadel (17) in ihrem Ruhezustand zu halten.

6. Mikronadelvorrichtung wie in einem der vorhergehenden Ansprüche beansprucht, **dadurch gekennzeichnet, dass** jede Hauptkammer (40, 112) so konfiguriert ist, dass sie Flüssigkeit aufnimmt, die durch sie hindurch von der Flüssigkeitsquelle (57) zu den entsprechenden ein oder mehreren Mikronadeln (17) fließt.

7. Mikronadelvorrichtung wie in einem der vorhergehenden Ansprüche beansprucht, **dadurch gekennzeichnet, dass** die Hauptkammer (40, 112) so konfiguriert ist, dass sie mit einem Flüssigkeitsleitungsmittel (52) in Verbindung steht, das so konfiguriert ist, dass es eine Flüssigkeit von einer entfernten Quelle (51) an die Hauptkammer (40, 112) leitet.

8. Mikronadelvorrichtung wie in Anspruch 7 beansprucht, **dadurch gekennzeichnet, dass** das Flüssigkeitsleitungsmittel (52) eine längliche Flüssigkeitsleitungsnadel mit einer Flüssigkeitsaufnahmebohrung (55) umfasst, die sich durch diese hindurch bis zu einem distalen Ende (56) dieser erstreckt.

9. Mikronadelvorrichtung wie in Anspruch 8 beansprucht, **dadurch gekennzeichnet, dass** das Trägerelement (5) oder das Betätigungsmittel (30) einen Flüssigkeitsleitungsnadelaufnahmekanal (35, 120) zur Aufnahme der Flüssigkeitsleitungsnadel (52) durch diesen hindurch zur Hauptkammer (40, 112) umfasst.

10. Mikronadelvorrichtung wie in Anspruch 9 beansprucht, **dadurch gekennzeichnet, dass** jede Hauptkammer (40, 112) von dem Flüssigkeitsleitungsnadelaufnahmekanal (35, 120) durch ein erstes Dichtungselement (29, 118) getrennt ist, und jedes erste Dichtungselement (39, 118) derart konfiguriert ist, dass es die Flüssigkeitsleitungsnadel (52) abdichtend durch dieses hindurch zu der entsprechenden der Hauptkammern (40, 112) aufnimmt.

11. Mikronadelvorrichtung wie in Anspruch 10 beansprucht, **dadurch gekennzeichnet, dass** jedes erste Dichtungselement (39, 118) ein selbstabdichtendes Dichtungselement umfasst, das so konfiguriert ist, dass es sich beim Herausziehen der Flüssigkeitsleitungsnadel (52) selbst wieder abdichtet.

12. Mikronadelvorrichtung wie in einem der vorhergehenden Ansprüche beansprucht, **dadurch gekennzeichnet, dass** jede Hauptkammer (40, 112) in dem Betätigungsmittel (30) angeordnet ist, und das Betätigungsmittel (30) mit dem Trägerelement (5) ausrichtbar ist, um die mindestens eine Hauptkammer (40, 112) mit entsprechenden der einen oder mehreren Mikronadeln (17) auszurichten, um die Hauptkammer (40, 112) mit einer Fluidaufnahmebohrung (20) jeder Mikronadel (17) zu verbinden, die mit der Hauptkammer (40, 112) korrespondiert.

13. Mikronadelvorrichtung wie in einem der vorhergehenden Ansprüche beansprucht, ferner umfassend ein Abschirmmittel (24, 98) zum Schützen der Mikronadeln (17).

14. Mikronadelvorrichtung wie in Anspruch 13 beansprucht, **dadurch gekennzeichnet, dass** das Abschirmmittel (24, 98) eine röhrenförmige Abschirmung (24, 98) umfasst, die eine Trägerelementaufnahmebohrung (25) aufweist, die sich durch dieses erstreckt, um das Trägerelement (5) und die Mikronadeln (17) darin aufzunehmen, wenn sich die Mikronadeln im Ruhezustand befinden.

15. Mikronadelvorrichtung wie in einem der vorhergehenden Ansprüche beansprucht zur Verwendung in einem Verfahren zur Behandlung einer Stelle im Körper eines Menschen oder eines Tieres mit einer injizierbaren Flüssigkeit.

## Revendications

1. Dispositif de micro-aiguille comprenant un élément porteur (5) définissant un axe principal (15) s'étendant longitudinalement, au moins une micro-aiguille (17) définissant un axe d'aiguille (19), la au moins une micro-aiguille (17) étant portée sur l'élément porteur (5) avec l'axe d'aiguille (19) s'étendant sensiblement transversalement par rapport à l'axe principal (15) défini par l'élément porteur (5), et un moyen de fonctionnement (30) coopérant avec l'un des éléments porteurs (5) et l'au moins une micro-aiguille (17) pour pousser la micro-aiguille (17) transversalement vers l'extérieur par rapport à l'axe principal (15) d'un état de repos à un état d'engagement pour engager du tissu, **caractérisé en ce que** l'un de l'élément porteur (5) et du moyen de fonctionnement (30) est configuré pour accueillir un moyen de communication (35, 40, 112, 120) pour communiquer chaque micro-aiguille (17) avec une source (51) du fluide à délivrer à un sujet par l'intermédiaire du dispositif de micro-aiguille, et le moyen de communication (35,40,112,120) comprend au moins une chambre principale (40,112) située dans l'élément porteur (5) et le moyen de fonctionnement (30), chaque chambre principale (40,112) étant configurée pour accueillir le fluide à administrer à un sujet et pour communiquer avec l'une ou plusieurs des micro-aiguilles correspondantes.

2. Dispositif de micro-aiguille selon la revendication 1, **caractérisé en ce que** l'élément porteur (5) définit un alésage principal (14) s'étendant longitudinalement dans l'élément porteur (5) à partir d'une extrémité proximale (6) de celui-ci, le moyen de fonctionnement (30) pouvant être engagé dans l'alésage principal (14) défini par l'élément porteur (5) et étant configuré pour augmenter la section transversale de l'alésage principal (14) défini par l'élément porteur (5).

3. Dispositif de micro-aiguilles selon la revendication 1 ou 2, **caractérisé en ce que** le moyen de fonctionnement (30) comprend un organe de fonctionnement, l'organe de fonctionnement (30) ayant une section transversale indéformable.

4. Dispositif de micro-aiguilles selon l'une quelconque des revendications précédentes, **caractérisé en ce que** l'élément porteur (5) comprend une paire de coquilles (9).

5. Dispositif de micro-aiguille selon la revendication 4, en ce que les coquilles (9) sont poussées de façon résiliente l'une vers l'autre dans un état de repos pour retenir au moins une micro-aiguille (17) dans l'état de repos.

6. Dispositif de micro-aiguilles selon l'une quelconque des revendications précédentes, **caractérisé en ce que** chaque chambre principale (40,112) est configurée pour accueillir le fluide qui la traverse depuis la source de fluide (57) jusqu'à la ou les micro-aiguilles correspondantes (17).

7. Dispositif de micro-aiguilles selon l'une quelconque des revendications précédentes, **caractérisé en ce que** la chambre principale (40), 112) est configurée pour communiquer avec un moyen d'administration de fluide (52) configuré pour administrer un fluide à partir d'une source distante (51) à la chambre principale (40, 112).

8. Dispositif de micro-aiguille selon la revendication 7, **caractérisé par le fait que** le moyen d'administration de fluide (52) comprend une aiguille d'administration de fluide allongée ayant un alésage d'accueil de fluide (55) s'étendant à travers elle jusqu'à son extrémité distale (56).

9. Dispositif de micro-aiguilles selon la revendication 8, **caractérisé en ce que** l'un de l'élément porteur (5) et du moyen de fonctionnement (30) comprend un conduit d'accueil de l'aiguille d'administration de fluide (35,120) pour accueillir l'aiguille d'administration de fluide (52) à travers la chambre principale (40,112).

10. Dispositif de micro-aiguille selon la revendication 9, **caractérisé en ce que** chaque chambre principale (40,112) est isolée du conduit d'accueil de l'aiguille d'administration de fluide (35,120) par un premier élément d'étanchéité (29,118), et que chaque premier élément d'étanchéité (39,118) est configuré pour accueillir de manière étanche l'aiguille d'administration de fluide (52) à travers l'une des chambres principales correspondantes (40,112).

11. Dispositif de micro-aiguille selon la revendication 10, **caractérisé en ce que** chaque premier élément d'étanchéité (39,118) comprend un élément d'étanchéité auto-obturant configuré pour se refermer lors du retrait de l'aiguille d'administration de fluide (52).

12. Dispositif de micro-aiguilles selon l'une quelconque des revendications précédentes, **caractérisé en ce que** chaque chambre principale (40,112) est située dans le moyen de fonctionnement (30), et le moyen de fonctionnement (30) peut être aligné avec l'élément porteur (5) pour aligner au moins une chambre principale (40,112) avec une ou plusieurs des micro-aiguilles (17) correspondantes pour faire communiquer la chambre principale (40,112) avec un alésage d'accueil de fluide (20) de chaque micro-aiguille (17) correspondant à la chambre principale (40,112).

13. Dispositif de micro-aiguilles selon l'une quelconque des revendications précédentes, comprenant en outre un moyen de protection (24,98) pour protéger les micro-aiguilles (17).

14. Dispositif de micro-aiguille selon la revendication 13, **caractérisé en ce que** le moyen de protection (24,98) comprend une protection tubulaire (24,98) ayant un alésage d'accueil de l'élément porteur (25) s'étendant à travers elle pour accueillir l'élément porteur (5) et les micro-aiguilles (17) lorsque les micro-aiguilles sont à l'état de repos.

15. Dispositif de micro-aiguilles selon l'une quelconque des revendications précédentes, pour utilisation dans une méthode de traitement d'un site du corps humain ou animal avec un liquide injectable.
